# EUROPÄISCHE PATENTSCHRIFT

(11) **EP 1 601 302 B1**
(45) Veröffentlichungstag und Bekanntmachung des Hinweises auf die Patenterteilung: **12.10.2016**
(21) Anmeldenummer: 04705368.1
(22) Anmeldetag: 27.01.2004
(51) Int. Cl.: A61C 8/00, A61C 3/02, A61B 17/16

(54) **PILOTBOHRER, STUFENBOHRER UND BOHRERSET FÜR DIE DENTALIMPLANTOLOGIE**
PILOT DRILL, STEP DRILL AND DRILL SET FOR DENTAL IMPLANT TECHNOLOGY
FORET PILOTE, FORET ETAGE ET ENSEMBLE DE FORETS POUR LA TECHNIQUE D'IMPLANT DENTAIRE

(30) Priorität: 13.03.2003 CH 4022003
(43) Veröffentlichungstag der Anmeldung: 07.12.2005
(73) Patentinhaber: THOMMEN MEDICAL AG, 2540 Grenchen (CH)
(72) Erfinder: STOOP, Hans, CH-4442 Diepflingen (CH)
(74) Vertreter: Ullrich, Gerhard
(86) Internationale Anmeldenummer: PCT/CH2004/000042
(87) Internationale Veröffentlichungsnummer: WO 2004/080325

(56) Entgegenhaltungen:
- EP-A- 0 454 639
- WO-A-01/64125
- DE-U- 20 115 184
- US-A- 5 078 607
- US-A1- 2002 028 422

## Beschreibung

### Anwendungsgebiet der Erfindung

Die vorliegende Erfindung betrifft einen Pilotbohrer, einen Stufenbohrer und ein daraus gebildetes Bohrerset zur Verwendung in der Dentalimplantologie. Der Pilotbohrer dient zur Herstellung einer in einen humanen Kieferknochen einzubringenden sacklochförmigen Pilotbohrung als Vorbereitung für deren Vergrösserung zu einer Stufenbohrung, was mit einem oder - bei weiterer Vergrösserung der Stufenbohrung - mehreren unterschiedlichen Stufenbohrer geschieht. Die präparierte Stufenbohrung ist zur Aufnahme eines Dentalimplantats, vorzugsweise in Schraubenform, bestimmt. Bei schraubenförmigen Implantaten kann das vorbereitete Bohrloch vor der Applikation mit seinem Innengewinde versehen werden oder das Implantat ist selbst-schneidend, wodurch das Innengewinde mit dem Eindrehen des Implantats in den Kieferknochen geschnitten wird. Die hiesige Erfindung bezieht sich vorrangig auf Dentalimplantate in Schraubenform. Das eingeheilte Implantat bildet die Verankerung für eine aufzubauende Suprastruktur.

### Stand der Technik

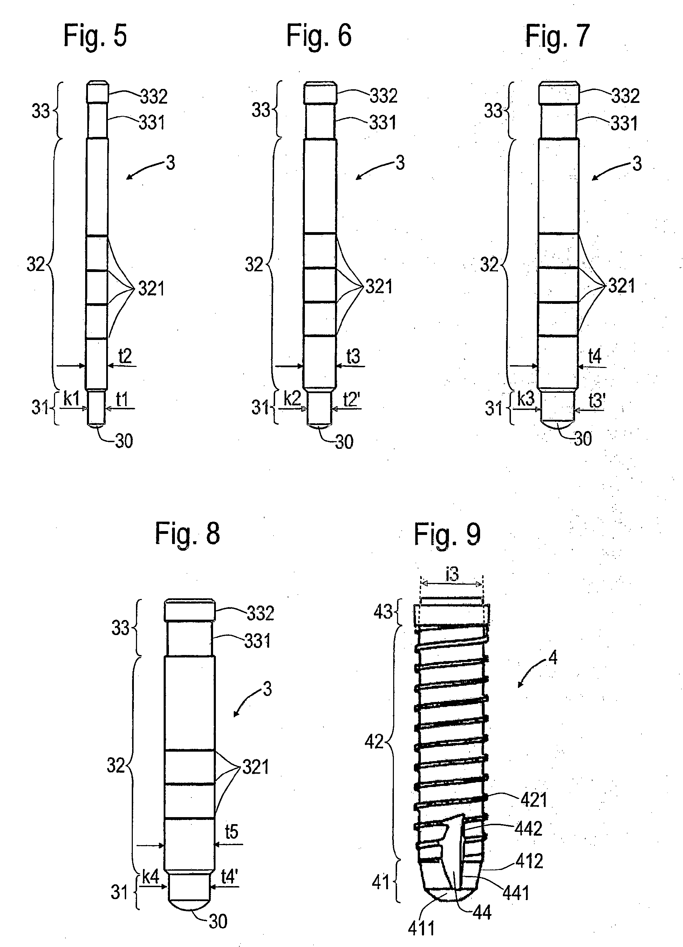

Für das Anfertigen der Aufnahmebohrung als Implantatbett sind verschiedene Lösungen bekannt. Bei SCHROEDER, A.; SUTTER, F.; BUSER, D.; KREKELER, G.: Oral Implantology. Georg Thieme Verlag Stuttgart, 2. Aufl. 1996, S. 153 ff., Fig. 7.42c, wird ein Bohrerset gezeigt, das aus einem Rosenbohrer und drei Spiralbohrern mit zunehmenden Querschnitten besteht. Der Rosenbohrer dient hierbei zum Markieren der Position auf der Kortikalis, wo die Bohrung eingebracht werden soll. Quasi übergangslos werden die Kortikalis und Spongiosa mit dem ersten Spiralbohrer aufgebohrt.

Die Wieland Dental + Technik GmbH & Co. KG, D-75179 Pforzheim, Deutschland, offeriert eine Bohrersequenz für ein selbst-schneidendes Schraubenimplantat mit Ø3.3mm und konischer Halspartie, bestehend aus folgenden Bohrinstrumenten:
a) einen Initialbohrer zum genauen Anbohren der Position;
b) einen Spiralbohrer Ø1.8mm. zur Präparation der Bohrung in voller Tiefe;
c) einen Stufenbohrer Ø1.8mm. / Ø2.5mm. zur Aufweitung der Bohrungsmündung auf Ø2.5mm;
d) einen Spiralbohrer Ø2.5mm. zur Aufweitung der Bohrung über die gesamte Tiefe auf Ø2.5mm; und
e) einen konischen Bohrer mit einer Führung Ø2.5mm. zur Aufweitung der Bohrungsmündung.

Bei der Applikation eines Implantats mit Ø5.5mm werden bis zu vier weitere Bohrer eingesetzt. Vor dem Einsatz des nächst grösseren Bohrers wird die bestehende Bohrung über einen kurzen Mündungsbereich auf den Durchmesser des nächst grösseren Durchmessers aufgeweitet, um eine optimale Zentrierung des nachfolgenden Bohrers zu gewährleisten. Oder die nachfolgenden Bohrer nehmen im Durchmesser nur geringfügig zu. Dies hat zur Folge, dass viele Bohrinstrumente und Arbeitsschritte nötig sind.

Die Implant Innovation Inc., USA, bietet ein Bohrerset an, wo auch vor dem Einsatz des nächst grösseren Bohrers die bestehende Bohrung über einen kurzen Mündungsbereich auf den nächst höheren Durchmesser aufgeweitet wird, um eine gute Zentrierung des nachfolgenden Bohrers zu gewährleisten. Dazu werden jeweils Stufenbohrer verwendet. Diese Stufenbohrer besitzen apikal eine runde Nase im Durchmesser des vorangehenden Bohrers, welche die Zentrierung in der Bohrung bewirkt und keine schneidende Funktion hat. Dieses System verlangt für jeden Bohrdurchmesser nach einem Stufenbohrer und dem korrespondierenden Spiralbohrer, so dass auch hier eine grössere Zahl von Bohrinstrumenten und Arbeitgängen anfallen.

Schliesslich ist eine Bohrer-Reihenfolge der FRIADENT GmbH, D-68229 Mannheim, Deutschland, bekannt. Z.B. für ein selbst-schneidendes 3-stufiges Schraubenimplantat Ø5.5mm werden folgende Bohrer verwendet:
a) ein Vorbohrer Ø2.mm zum genauen Anbohrung der Position und Vorgabe der Achsrichtung;
b) ein Rosenbohrer Ø3.4mm zur Aufweitung der Bohrungsmündung auf Ø3.4mm;
c) ein 3-stufiger Stufenfräser zur Präparation einer 3-stufigen Bohrung Ø3.4mm;
d) ein 3-stufiger Stufenfräser zur Präparation einer Bohrung Ø3.8mm;
e) ein 3-stufiger Stufenfräser zur Präparation einer Bohrung Ø4.5mm; und
f) ein 3-stufiger Stufenfräser zur Präparation einer Bohrung Ø5.5mm.

Durch die spezielle 3-stufige Geometrie des Implantats wurde hier ein System gewählt, bei welchem bereits nach der zweiten Bohrung die 3-stufige Form des Implantats aufbereitet und bei jedem weiteren Schritt diese Form vergrössert wird. Je nach Durchmesser des Implantats wird die Bohrung durch mehrfache Anwendung des Stufenfräsers aufgeweitet. Aufgrund der mehrstufigen Geometrie benötigt man jedoch für jede Implantatlänge einen speziellen Satz von Stufenfräsern. Das führt auch hier zu einer Instrumentenvielfalt und zudem sind 3-stufige Fräser deutlich teurer.

Die US 5,871,356 A offenbart die Verwendung eines herkömmlichen Pilotbohrers für den ersten Schritt bei der Herstellung eines Bettes zum Einsetzen eines Dentalimplantats und anschliessend für den zweiten Schritt zum Aufbohren des Implantatbettes einen Stufenbohrer sowie ferner ein im Implantatbett das Gewinde selbstschneidende Dentalimplantat in Gestalt eines Hohlzylinders. Der Stufenbohrer besitzt ein apikales Teilstück, dessen Durchmesser eine Grösse hat, welche zwischen dem lichten Innendurchmesser des apikalen Endes des Implantats und dem Aussendurchmesser an diesem Ende bemessen ist. An das apikale Teilstück des Stufenbohrers schliesst sich ein proximales Teilstück an, dessen Durchmesser eine Grösse hat, welche zwischen dem Aussendurchmesser des apikalen Endes des Implantats und dem Aussendurchmesser des Gewindes am Implantat bemessen ist. Vom proximalen Teilstück des Stufenbohrers erstreckt sich ein kegelstumpfförmiges Teilstück, welches zur Aussengeometrie des koronalen Endes des Implantats komplementär ist.

Bei allen erwähnten Bohrersequenzen wird anfangs eine meist kurze Bohrung in der Kortikalis angebracht, um die Position für den nachfolgenden Bohrer festzulegen, mit diesem dann die genaue Richtung vorzugeben und danach die Bohrung sukzessive aufzuweiten. Alle vorgestellten Bohrersysteme erfordern mehr Arbeitsschritte - Bohrvorgänge und Bohrerwechsel - als unterschiedliche Bohrdurchmesser anzubringen sind, was einen erhöhten Zeitbedarf verursacht, das operative Vorgehen verkompliziert, nach relativ vielen verschiedenen Instrumenten verlangt und auch die Gefahr von Fehlern erhöht.

### Aufgabe der Erfindung

Angesichts der bei den existenten Bohrwerkzeugen bestehenden Unvollkommenheiten, liegt die Aufgabe zugrunde, einen verbesserten Pilotbohrer zu schaffen. Eine weitere Aufgabe ist die Schaffung eines verbesserten Stufen-bohrers. Eine zusätzliche Aufgabe besteht darin, aus dem Pilot- und Stufenbohrer ein mehrteiliges, vorteilhaft einsetzbares Bohrerset vorzuschlagen. Hierbei ist davon auszugehen, dass die eingebrachte Bohrung örtlich sehr präzise am planungsgerechten Ort die Kortikalis durchstösst, um eine korrekte Position des Implantates und somit des späteren Zahnersatzes zu gewährleisten. Die Bohrungsrichtung muss exakt ausgerichtet sein, um die später auf den Zahnersatz einwirkenden Belastungen optimal aufzunehmen. Bei der Präparation des Implantatbetts soll der Kieferknochen möglichst gering beansprucht werden. Die Präparation der Bohrung muss mit wenigen Handgriffen einfach und zeitsparend erfolgen können, dazu soll nur eine geringe Anzahl von Instrumenten erforderlich sein. Schliesslich gilt es die insgesamt entstehenden Kosten tief zu halten.

### Übersicht über die Erfindung

Die Aufgabe wird gelöst durch den Gegenstand der unabhängigen Ansprüche 1, 5 und 9, wobei die US 5,871,356 A deren zweiteilige Form veranlasst. Vorteilhafte Ausführungsformen gehen aus den abhängigen Ansprüchen hervor.

Zur Vorbereitung des Implantatbettes für die Aufnahme eines Dentalimplantats - als einzubringende sacklochförmige Stufenbohrung im humanen Kieferknochen - ist ein Pilotbohrer vorgesehen. Der Pilotbohrer hat an seinem apikalen Ende eine Pilotspitze mit Spitzenschneiden. Von der Pilotspitze in Richtung des koronalen Endes des Pilotbohrers erstreckt sich eine Pilotführung, oberhalb derer ein Bohrhals liegt, welcher einen grösseren Bohrerdurchmesser aufweist als der Bohrerdurchmesser der Pilotführung. An den Bohrhals schliesst sich ein Bohrschaft an, und als koronales Ende besitzt der Pilotbohrer eine standardisierte Dentalkupplung, wie sie für zahnärztliche Handstücke an elektrischen Bohrmaschinen üblich ist. Seitlich an der Pilotführung liegt zumindest eine Führungsschneide. Im Übergang von der Pilotführung zum Bohrhals befindet sich eine Stufe mit zumindest einer Stufenschneide. Entlang des Pilotbohrers erstreckt sich zumindest eine Spiralnut und eine daran angrenzende Fase. Das Charakteristische des Pilotbohrers besteht zunächst darin, dass die Spitzenschneiden an der Pilotspitze scharf ausgebildet und zentrums-schneidend sind und sich Anschliffe von den Spitzenschneiden aufwärts der Pilotführung erstrecken. Die Stufenschneiden an der Stufe sind schneidend ausgebildet, während die Führungsschneiden stumpf, also nicht-schneidend gestaltet sind.

Die nachfolgenden Merkmale stellen vorteilhafte Ausführungsformen der Erfindung dar: Der Bohrhals mit der Fase ist schwach schneidend ausgebildet ist. Die Pilotführung hat eine Länge im Bereich von 1.0mm bis 4.0mm, z.B. 3.0mm. Vorzugsweise ist der Pilotbohrer zweischneidig ausgebildet und besitzt somit jeweils zwei Spitzenschneiden, Anschliffe, Führungsschneiden, Spiralnuten, Fasen und Stufenschneiden. Der Bohrhals hat mindestens die Länge der Einsetztiefe des zu applizierenden Implantats. Die Pilotführung weist den Durchmesser im Bereich von 1.5mm auf und der Bohrhals hat den Durchmesser im Bereich von 2.0mm. Der zwischen den Spitzenschneiden liegende Spitzenwinkel ist kleiner als 90°, vorzugsweise liegt er im Bereich von 80°. Die Spiralnuten erstrecken sich durchgängig vom koronalen Ende des Bohrhalses bis in die Pilotspitze, wobei an der Pilotführung die Spiralnuten durch den geringeren Durchmesser nur mehr einen Anteil ihres vollen Querschnitts, wie er am Bohrhals vorhanden ist, aufweisen. Am Bohrhals sind zur Kontrolle der Eindringtiefe des Pilotbohrers mehrere sichtbare Tiefenmarkierungen in gleichen oder ungleichen Abständen angebracht.

Die Pilotführung mit der Pilotspitze sind dazu bestimmt, die Position der zu erzeugenden Stufenbohrung mit dem Einbringen eines Ansatzes einer Pilotbohrung durch die Kortikalis des Kieferknochen festzulegen, wobei der Ansatz aus einer Pilotbohrungsführung und einer Pilotbohrungsspitze besteht. Die Stufe ist dazu bestimmt, nach durchdrungener Kortikalis - mit Fertigstellung der Pilotbohrungsführung und -spritze - einen spürbar erhöhten Bohrwiderstand zu generieren, so dass der Chirurg bei diesem Anzeichen die angesetzte Bohrungsrichtung überprüfen kann. Die stumpfen Führungsschneiden ermöglichen, ohne Aufweitung der Pilotbohrungsführung die Bohrungsrichtung innerhalb eines kegelförmigen Korrekturbereichs zu korrigieren. Der Bohrhals mit seiner Dimensionierung ist dazu bestimmt, die Pilotbohrung in der endgültigen Tiefe zu erstellen.

Für die Vergrösserung einer zuvor in einen humanen Kieferknochen eingebrachten sacklochförmigen Pilotbohrung zu einer Stufenbohrung bzw. für die weitere Vergrösserung einer vorhandenen Stufenbohrung zu einer nochmals vergrösserten Stufenbohrung als Aufnahme für ein Dentalimplantat ist ein Stufenbohrer vorgesehen. Der Stufenbohrer hat eine Stufenspitze, die am apikalen Ende des Stufenbohrers liegt und mit Spitzenschneiden versehen ist. Eine Stufenführung erstreckt sich von der Stufenspitze in Richtung des koronalen Endes des Stufenbohrers. Oberhalb der Stufenführung liegt ein Bohrhals, der einen grösseren Bohrerdurchmesser aufweist als der Bohrerdurchmesser der Stufenführung. An den Bohrhals schliesst sich ein Bohrschaft an, und am koronalen Ende des Stufenbohrers liegt eine standardisierte Dentalkupplung zur Adaption in einem zahnärztlichen Handstück einer elektrischen Bohrmaschine. Der Stufenbohrer hat zumindest eine seitlich an der Stufenführung liegende Führungsschneide. Am Übergang von der Stufenführung zum Bohrhals ist eine Stufe mit zumindest einer Stufenschneide ausgebildet. Über den Stufenbohrer erstreckt sich zumindest eine Spiralnut und eine daran angrenzende Fase. Das Charakteristische des Stufenbohrers besteht zunächst darin, dass die Spitzenschneiden an der Stufenspitze scharf ausgebildet sind und sich Anschliffe von den Spitzenschneiden aufwärts der Stufenführung erstrecken. Die Stufenschneiden an der Stufe sind schneidend ausgebildet, während die Führungsschneiden stumpf, also nicht-schneidend sind.

Die nachfolgenden Merkmale stellen vorteilhafte Ausführungsformen der Erfindung dar: Der Bohrhals mit der Fase ist schwach schneidend ausgebildet. Die Stufenführung hat eine Länge im Bereich von 2.0mm. Vorzugsweise ist der Stufenbohrer dreischneidig ausgebildet ist und weist somit jeweils drei Spitzenschneiden, Anschliffe, Führungsschneiden, Spiralnuten, Fasen und Stufenschneiden auf. Der Bohrhals hat mindestens die Länge der Einsetztiefe des zu applizierenden Implantats. Die Stufenführung verschiedener, nämlich *erster, zweiter* und *dritter* Stufenbohrer hat einen Durchmesser im Bereich von 2.0mm, 2.8mm bzw. 3.5mm und der Bohrhals dieser *ersten, zweiten* und *dritten* Stufenbohrer hat den zugehörigen Durchmesser im Bereich von 2.8mm, 3.5mm bzw. 4.3mm. Der zwischen den Spitzenschneiden sich aufspannende Spitzenwinkel ist grösser als 90°, vorzugsweise liegt er im Bereich von 120°.

Die Spiralnuten erstrecken sich durchgängig vom koronalen Ende des Bohrhalses bis in die Stufenspitze, wobei an der Stufenführung die Spiralnuten durch den geringeren Durchmesser nur mehr einen Anteil ihres vollen Querschnitts, wie er am Bohrhals vorhanden ist, aufweisen. Zur Überprüfung der Eindringtiefe sind am Bohrhals mehrere sichtbare Tiefenmarkierungen in gleichen oder ungleichen Abständen angebracht. Die Stufenführung mit der Stufenspitze und den stumpfen Führungsschneiden ist dazu bestimmt, den Stufenbohrer beim Ansetzen in die Pilotbohrung bzw. Stufenbohrung zu zentrieren und beim Vortrieb entlang der Pilotbohrung bzw. der Stufenbohrung zentriert zu führen. Die Stufe mit den Stufenschneiden ist dazu bestimmt, die Pilotbohrung mit den vorherigen Durchmessern auf neue Durchmesser aufzuweiten bzw. die Stufenbohrung mit den vorherigen Durchmessern auf die neuen Durchmesser aufzuweiten.

Zur Vorbereitung und Erstellung eines Implantatbettes für die Aufnahme eines Dentalimplantats in einer im humanen Kieferknochen einzubringenden sacklochförmige Stufenbohrung ist ein Bohrerset vorgesehen, das zunächst aus einem vorbeschriebenen Pilotbohrers zum Anbringen einer Pilotbohrung besteht. Zum Bohrerset gehört ferner zumindest ein *erster* vorbeschriebener Stufenbohrer für die Vergrösserung der vorhandenen Pilotbohrung zu einer Stufenbohrung. Das Bohrerset ergänzt sich mit einem optionalen *zweiten* vorbeschriebenen Stufenbohrer für die zweite Vergrösserung einer vorhandenen Stufenbohrung zu einer nochmals vergrösserten Stufenbohrung. Schliesslich kann zum Bohrerset ein optionaler *dritter* vorbeschriebener Stufenbohrer für die dritte Vergrösserung der bereits zweifach vergrösserten Stufenbohrung zu einer letztmalig vergrösserten Stufenbohrung gehören.

### Kurzbeschreibung der beigefügten Zeichnungen

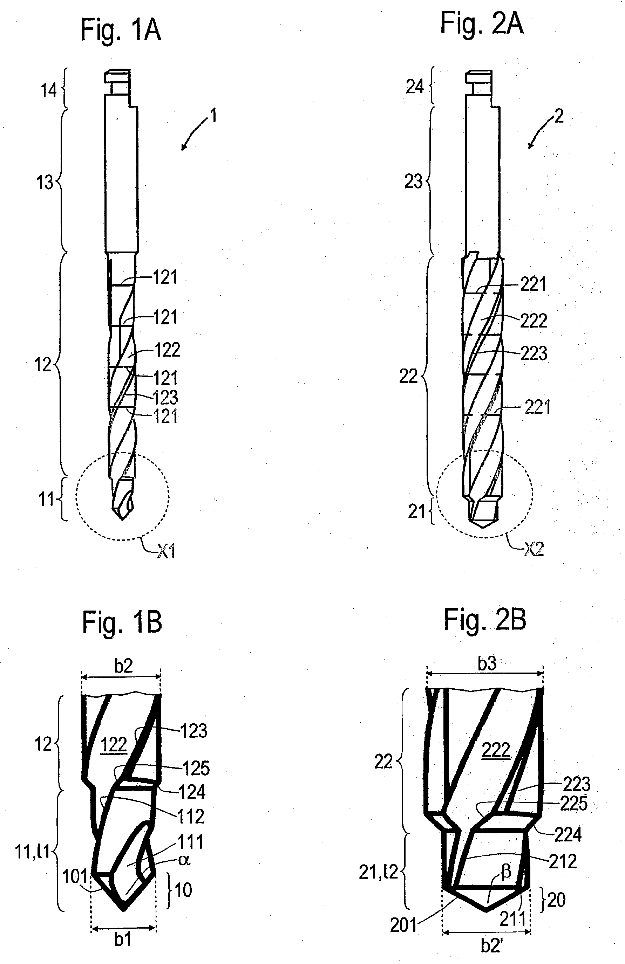

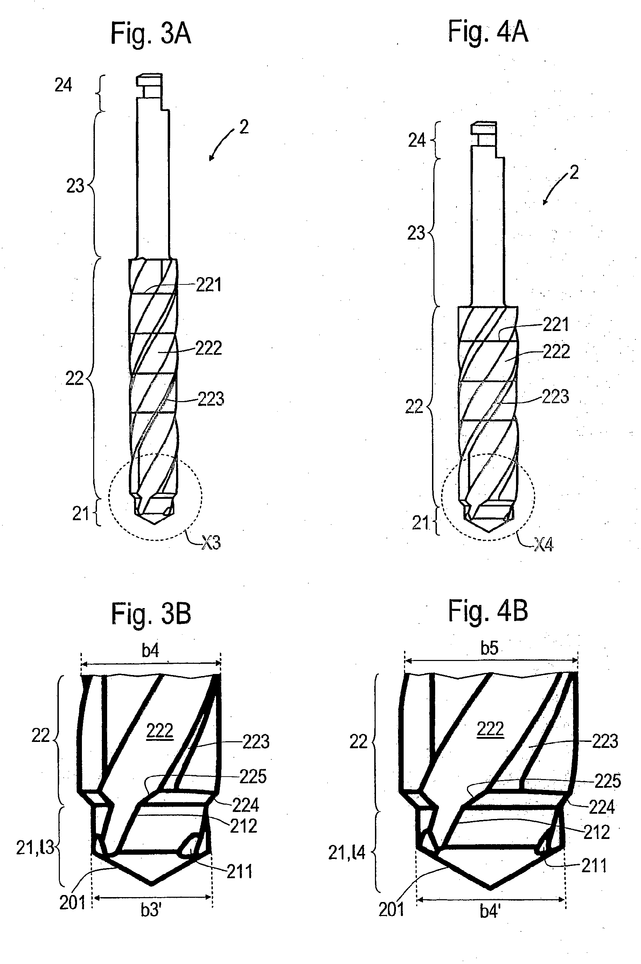

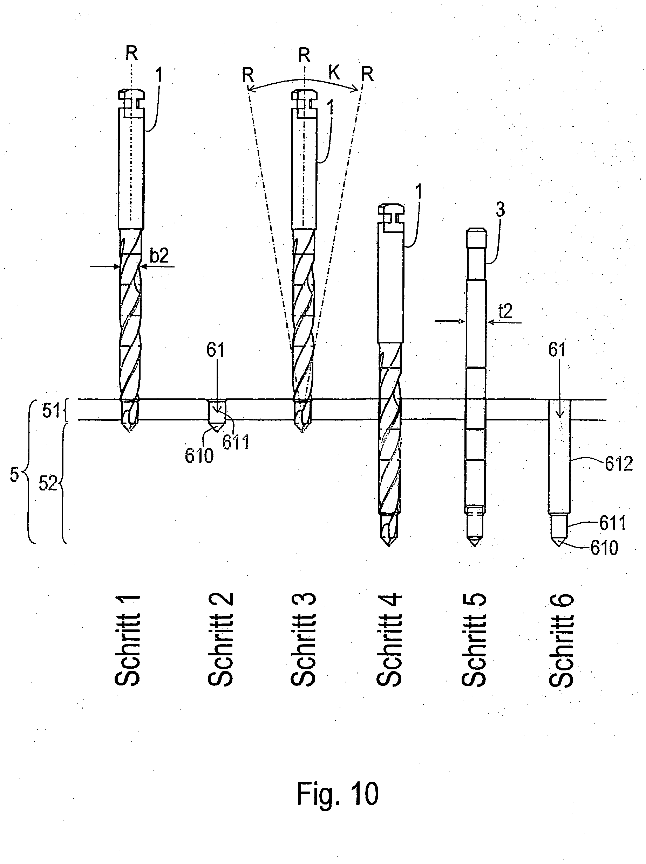

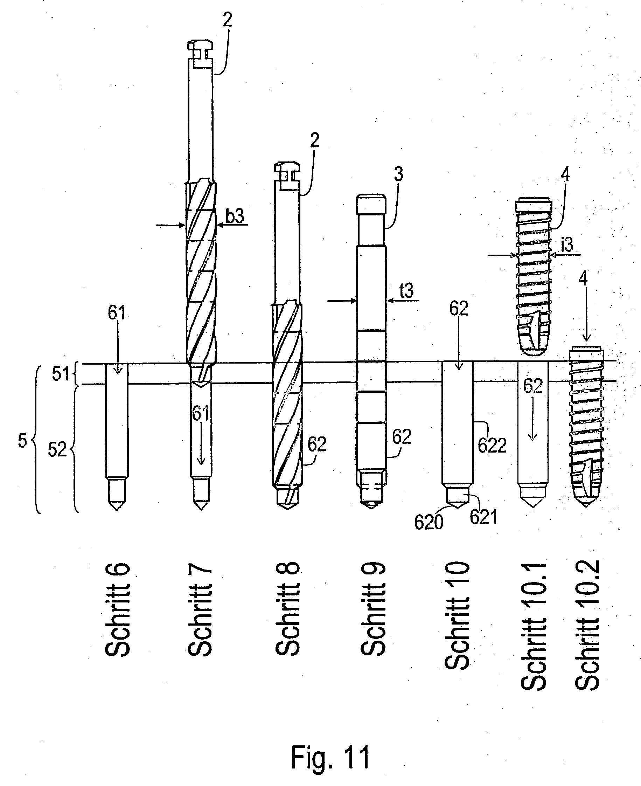

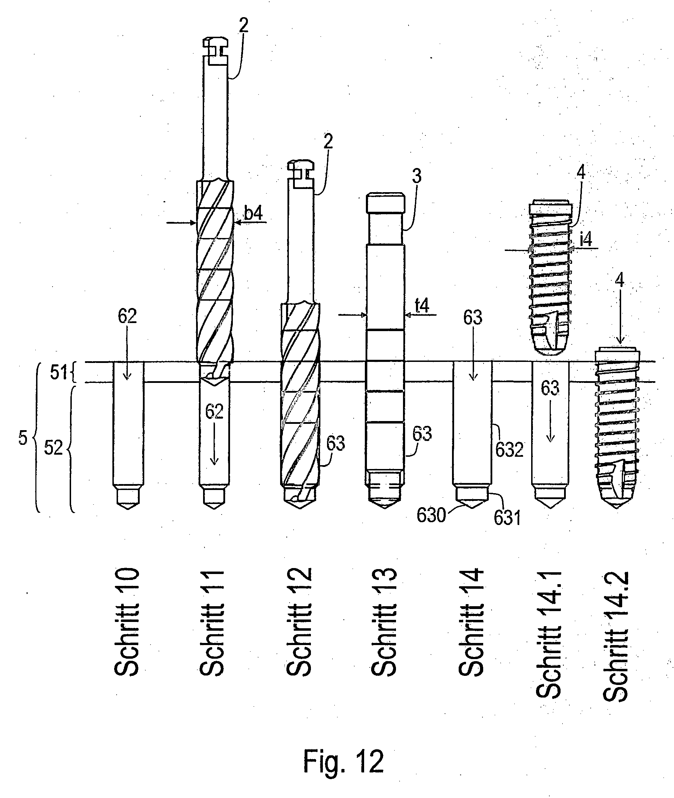

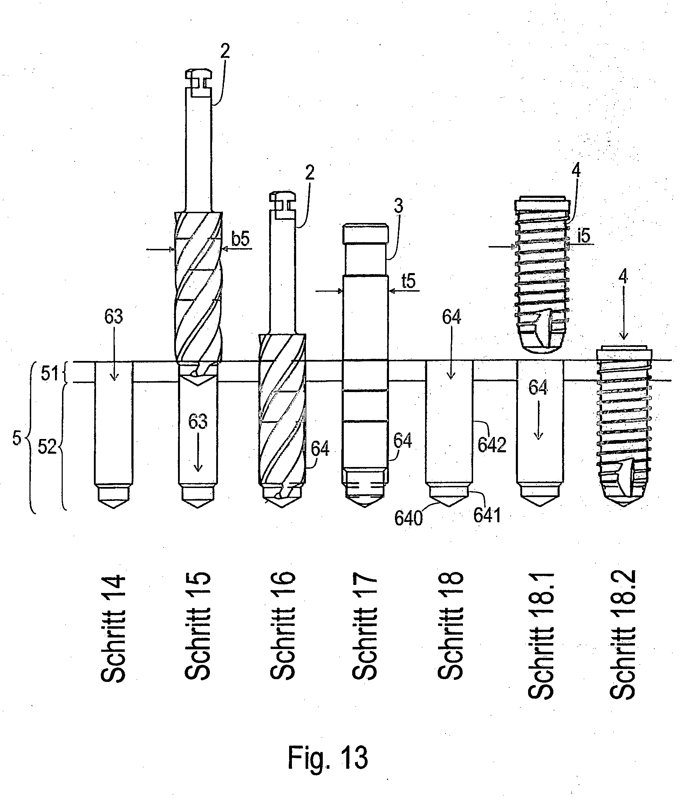

Mit Bezug auf die beiliegenden Zeichnungen erfolgt nachstehend die detaillierte Beschreibung eines Ausführungsbeispiels der erfindungsgemässen Anordnung. Es zeigen:
- Figur 1A:: einen erfindungsgemässen Pilotbohrer;
- Figur 1B:: das vergrösserte Detail X1 aus Figur 1A mit der Spitze des Pilotbohrers;
- Figur 2A:: einen erfindungsgemässen *ersten* Stufenbohrer mit dem kleinsten Durchmesser;
- Figur 2B:: das vergrösserte Detail X2 aus Figur 2A mit der Spitze des *ersten* Stufenbohrers;
- Figur 3A:: einen *zweiten* Stufenbohrer mit mittlerem Durchmesser;
- Figur 3B:: das vergrösserte Detail X3 aus Figur 3A mit der Spitze des *zweiten* Stufenbohrers;
- Figur 4A:: einen *dritten* Stufenbohrer mit grösstem Durchmesser;
- Figur 4B:: das vergrösserte Detail X3 aus Figur 3A mit der Spitze des *dritten* Stufenbohrers;
- Figur 5:: eine Tiefenlehre für den Pilotbohrer gemäss Figur 1A;
- Figur 6:: eine Tiefenlehre für den *ersten* Stufenbohrer gemäss Figur 2A;
- Figur 7:: eine Tiefenlehre für den *zweiten* Stufenbohrer gemäss Figur 3A;
- Figur 8:: eine Tiefenlehre für den *dritten* Stufenbohrer gemäss Figur 4A;
- Figur 9:: ein an sich bekanntes Dentalimplantat zum Einsetzen in eine mit dem *ersten* Stufenbohrer gemäss Figur 2A erzeugte Bohrung;
Figuren 10 bis 13:
das prinzipielle operative Handling des Bohrersets, beginnend bei der Ausgangssituation gemäss Figur 10, Schritt 1, bis zur Erstellung der fertigen Bohrung gemäss Figur 11, Schritt 10 (für den kleinsten Implantatdurchmesser); Figur 12, Schritt 14 (für den mittleren Implantatdurchmesser); Figur 13, Schritt 18 (für den grössten Implantatdurchmesser);
Figur 10:
- *Schritt 1-*: Erzeugen der Pilotführung in der Kortikalis mit dem Pilotbohrers gemäss Figur 1A;
- *Schritt 2 -*: optische Kontrolle der Position der erzeugten Pilotführung;
- *Schritt 3 -*: Einführen des Pilotbohrers in die vorhandene Pilotführung und definitive Bestimmung der Bohrungsrichtung;
- *Schritt 4 -*: Erzeugen der Pilotbohrung mit voller Böhrtiefe;
- *Schritt 5-*: Kontrolle der Bohrtiefe mit der Tiefenlehre gemäss Figur 5 für den Pilotbohrer;
- *Schritt 6 -*: optische Kontrolle der erzeugten Pilotbohrung;
Figur 11:
- *Schritt 6 -*: optische Kontrolle der erzeugten Pilotbohrung (Übernahme von Figur 10, Schritt 6);
- *Schritt 7 -*: Einführen des *ersten* Stufenbohrers gemäss Figur 2A in die vorhandene Pilotbohrung;
- *Schritt 8 -*: Erzeugen der *ersten* Stufenbohrung auf die volle Bohrtiefe;
- *Schritt 9 -*: Kontrolle der Bohrtiefe mit der Tiefenlehre gemäss Figur 6 für den *ersten* Stufenbohrer;
- *Schritt 10 -*: optische Kontrolle der erzeugten *ersten* Stufenbohrung;
- *Schritt 10.1 -*: Option: Einsetzen des Implantats mit dem kleinsten Durchmesser gemäss Figur 9;
- *Schritt 10.2 -*: Option: eingesetztes Implantat mit dem kleinsten Durchmesser gemäss Figur 9;
Figur 12:
- *Schritt 10*: *-* optische Kontrolle der angebrachten *ersten* Stufenbohrung (Übernahme von Figur 11, Schritt 10);
- *Schritt 11*: *-* Einführen des *zweiten* Stufenbohrers gemäss Figur 3A in die vorhandene *erste* Stufenbohrung;
- *Schritt 12*: *-* Erzeugen der *zweiten* Stufenbohrung auf die volle Bohrtiefe;
- *Schritt 13*: *-* Kontrolle der Bohrtiefe mit der Tiefenlehre gemäss Figur 7 für den *zweiten* Stufenbohrer;
- *Schrift 14*: *-* optische Kontrolle der angebrachten *zweiten* Stufenbohrung;
- *Schritt 14.1*: *-* Option: Einsetzen des Implantats mit dem mittleren Durchmesser;
- *Schritt 14.2*: *-* Option: eingesetztes Implantat mit dem mittleren Durchmesser;
Figur 13:
- *Schritt 14*: *-* optische Kontrolle der angebrachten *zweiten* Stufenbohrung (Übernahme von Figur 12, Schritt 14);
- *Schritt 15*: *-* Einführen des *dritten* Stufenbohrers gemäss Figur 4A in die vorhandene *zweite* Stufenbohrung;
- *Schritt 16*: *-* Erzeugen der *dritten* Stufenbohrung auf die volle Tiefe;
- *Schritt 17*: *-* Kontrolle der Bohrtiefe mit der Tiefenlehre gemäss Figur 8 für den *dritten* Stufenbohrer;
- *Schritt 18*: *-* Optische Kontrolle der erzeugten *dritten* Stufenbohrung;
- *Schritt 18.1*: *-* Einsetzen des Implantats mit dem grössten Durchmesser;
- *Schritt 18.2*: *-* eingesetztes Implantat mit dem grössten Durchmesser;

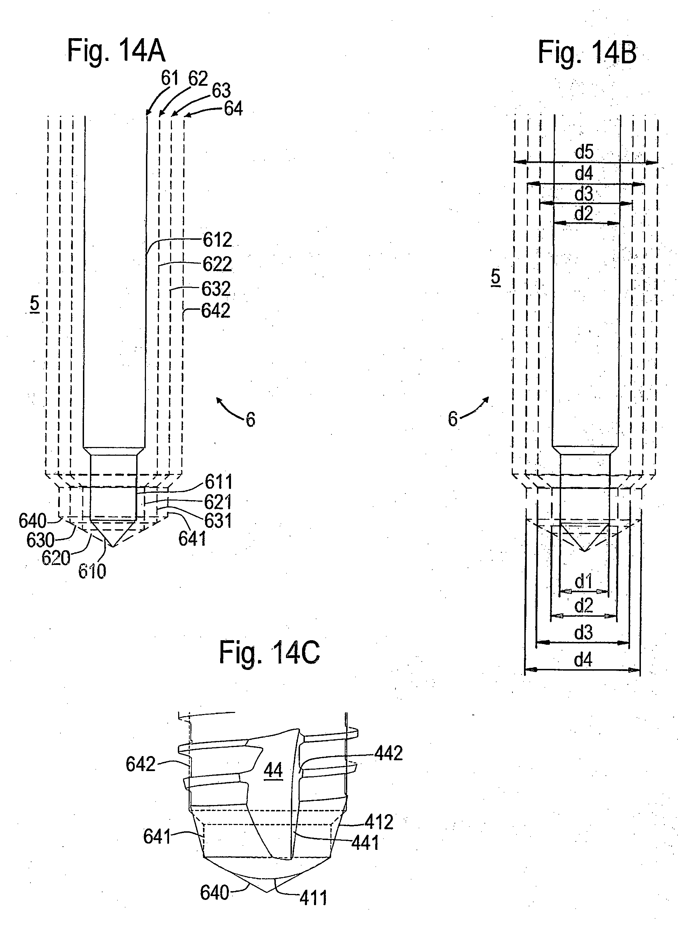

- Figur 14A:: eine Darstellung aller Bohrquerschnitte übereinander;
- Figur 14B:: eine Darstellung aller Bohrquerschnitte mit Bezeichnung der zueinander passenden Durchmesser von Führung und Hals; und
- Figur 14C:: die Spitze des Implantats mit dem grössten Durchmesser im Bohrquerschnitt des *dritten* Stufenbohrers liegend.

### Ausführungsbeispiele

Für die gesamte weitere Beschreibung gilt folgende Festlegung: Sind in einer Figur zum Zweck zeichnerischer Eindeutigkeit Bezugsziffern enthalten, aber im unmittelbar zugehörigen Beschreibungstext nicht erläutert, so wird auf deren Erwähnung in vorangehenden Figurenbeschreibungen Bezug genommen. Im Interesse der Übersichtlichkeit wird auf die wiederholte Bezeichnung von Bauteilen in nachfolgenden oder derselben Figuren zumeist verzichtet, sofern zeichnerisch eindeutig erkennbar ist, dass es sich um "wiederkehrende" Bauteile handelt.

### Figuren 1A und 1B

Der Pilotbohrer **1** dient für die Vorbereitung einer in einen Kieferknochen einzubringenden sacklochförmigen Stufenbohrung zur Aufnahme eines Dentalimplantats. Am apikalen Ende des Pilotbohrers **1** befindet sich die Pilotspitze **10**, mit den daran angeordneten Spitzenschneiden **101,** welche den Spitzenwinkel α einschliessen. Eine Pilotführung **11** erstreckt sich von der Pilotspitze **10** in Richtung des koronalen Endes des Pilotbohrers **1.** Oberhalb der Pilotführung **11** liegt der Bohrhals **12,** der einen grösseren Bohrerdurchmesser **b2** aufweist als der Bohrerdurchmesser **b1** der Pilotführung **11.** An den Bohrhals **12** schliesst sich der Bohrschaft **13** an, und am koronalen Ende des Pilotbohrer **1** ist eine standardisierte Dentalkupplung **14** vorgesehen, die zur Aufnahme in einem zahnärztlichen Handstück dient, wie es an Bohrmaschinen typisch ist. Vorhanden sind zumindest eine seitlich an der Pilotführung **11** liegende Führungsschneide **112,** eine Stufe **124 -** als Übergang von der Pilotführung **1** zum Bohrhals **12** -, zumindest eine Stufenschneide **125** an der Stufe **124** und zumindest eine Spiralnut **122** sowie eine daran angrenzende Fase **123.**

Die Spitzenschneiden **101** an der Pilotspitze **10** sind scharf ausgebildet und zentrums-schneidend. Von den Spitzenschneiden **101** erstrecken sich Anschliffe **111** aufwärts der Pilotführung **11.** Die Stufenschneiden **125** an der Stufe **124** sind schneidend ausgebildet, während die Führungsschneiden **112** stumpf, nicht-schneidend sind. Der gesamte Bohrhals **12** mit der Fase **123** ist schwach schneidend ausgebildet. Die Pilotführung **11** weist eine Länge **l1** im Bereich von 1.0mm bis 4.0mm auf.

Vorzugsweise ist der Pilotbohrer **1** zweischneidig ausgebildet und besitzt somit jeweils zwei Spitzenschneiden **101,** Anschliffe **111,** Führungsschneiden **112,** Spiralnuten **122,** Fasen **123** und Stufenschneiden **125.** Der Bohrhals **12** hat mindestens die Länge der Einsetztiefe des zu applizierenden Implantats, und die Pilotführung **11** hat die Länge **l1** von 3.0mm. Die Pilotführung **11** weist den Durchmesser **b1** im Bereich von 1.5mm auf und der Bohrhals **12** besitzt den Durchmesser **b2** im Bereich von 2.0mm. Der zwischen den Spitzenschneiden **101** liegende Spitzenwinkel α ist kleiner als 90°; vorzugsweise liegt α im Bereich von 80°. Die Spiralnuten **122** erstrecken sich durchgängig vom koronalen Ende des Bohrhalses **12** bis in die Pilotspitze **10.** An der Pilotführung **11** weisen die Spiralnuten **122** durch den geringeren Durchmesser **b1** nur mehr einen Anteil ihres vollen Querschnitts auf, wie er am Bohrhals **12** vorhanden ist. Zur Kontrolle der Bohrtiefe während des Bohrvorgangs sind am Bohrhals **12** mehrere sichtbare Tiefenmarkierungen **121** in gleichen oder ungleichen Abständen angebracht.

### Figuren 2A bis 4B

In dieser Figurenfolge werden gezeigt:
- ein *erster* Stufenbohrer **2** mit dem kleinsten Durchmesser (Figuren 2A und 2B),
- ein *zweiter* Stufenbohrer **2** mit mittlerem Durchmesser (Figuren 3A und 3B), und
- ein *dritter* Stufenbohrer **2** mit grösstem Durchmesser (Figuren 4A und 4B).

Ein Stufenbohrer **2** dient für die Vergrösserung einer in einem Kieferknochen vorhandenen sacklochförmigen Pilotbohrung zu einer Stufenbohrung bzw. für die weitere Vergrösserung einer vorhandenen Stufenbohrung zu einer nochmals vergrösserten Stufenbohrung als Aufnahme für ein Dentalimplantat. Am apikalen Ende des Stufenbohrers **2** liegt die Stufenspitze **20** mit den Spitzenschneiden **201.** Von der Stufenspitze **20** in Richtung des koronalen Endes des Stufenbohrers **2** erstreckt sich die Stufenführung **21.** Oberhalb der Stufenführung **21** liegt der Bohrhals **22,** welcher einen grösseren Bohrerdurchmesser **b3,b4,b5** aufweist als der Bohrerdurchmesser **b2',b3',b4'** der Stufenführung **21.** Oberhalb des Bohrhalses **22** liegt der Bohrschaft **23,** an den sich als koronales Ende eine standardisierte Dentalkupplung **24** anschliesst. Vorhanden sind zumindest eine seitlich an der Stufenführung **21** liegende Führungsschneide **212,** eine Stufe **224 -** als Übergang von der Stufenführung **21** zum Bohrhals **22** -, zumindest eine Stufenschneide **225** an der Stufe **224** und zumindest eine Spiralnut **222** mit einer daran angrenzenden Fase **223.** Die Spitzenschneiden **201** an der Stufenspitze **20** sind scharf ausgebildet. Von den Spitzenschneiden **201** erstrecken sich Anschliffe **211** aufwärts der Stufenführung **21.** Die Stufenschneiden **225** an der Stufe **224** sind schneidend ausgebildet, während die Führungsschneiden **212** stumpf, nicht-schneidend ausgebildet sind. Der gesamte Bohrhals **22** mit der Fase **223** ist schwach schneidend ausgebildet. Die Stufenführung **21** des *ersten, zweiten* und *dritten* Stufenbohrers **2** hat eine einheitliche Länge **l2,l3,l4** im Bereich von 2.0mm.

Vorzugsweise ist der Stufenbohrer **2** dreischneidig ausgebildet und weist somit jeweils drei Spitzenschneiden **201,** Anschliffe **211,** Führungsschneiden **212,** Spiralnuten **222,** Fasen **223** und Stufenschneiden **225** auf. Der Bohrhals **22** hat mindestens die Länge der Einsetztiefe des zu applizierenden Implantats. Die Stufenführung **21** am *ersten* Stufenbohrer **2** weist den Durchmesser **b2'** im Bereich von 2.0mm auf, während der zugehörige Bohrhals **22** den Durchmesser **b3** im Bereich von 2.8mm hat. Die Stufenführung **21** am *zweiten* Stufenbohrer **2** hat den Durchmesser **b3'** im Bereich von 2.8mm, wobei dessen Bohrhals **22** den Durchmesser **b4** im Bereich von 3.5mm aufweist. Schliesslich hat die Stufenführung **21** am *dritten* Stufenbohrer **2** den Durchmesser **b4'** im Bereich von 3.5mm und dessen Bohrhals **22** besitzt den Durchmesser **b5** im Bereich von 4.3mm.

Der zwischen den Spitzenschneiden **201** liegende Spitzenwinkel β ist grösser als 90°, vorzugsweise liegt β im Bereich von 120°. Die Spiralnuten **222** erstrecken sich durchgängig vom koronalen Ende des Bohrhalses **22** bis in die Stufenspitze **20.** Durch den geringeren Durchmesser **b2',b3',b4'** an der Stufenführung **21** nehmen die Spiralnuten **222** nur mehr einen Anteil ihres vollen Querschnitts ein, wie er am Bohrhals **22** vorhanden ist. Wiederum zur Kontrolle der Bohrtiefe während des Bohrvorgangs sind am Bohrhals **22** mehrere sichtbare Tiefenmarkierungen **221** in gleichen oder ungleichen Abständen angebracht.

### Figuren 5 bis 8

In dieser Figurenfolge werden gezeigt:
- eine Tiefenlehre **3** für den Pilotbohrer **1** (Figur 5),
- eine Tiefenlehre **3** für den *ersten* Stufenbohrer **2** (Figur 6),
- eine Tiefenlehre **3** für den *zweiten* Stufenbohrer **2** (Figur 7), und
- eine Tiefenlehre **3** für den *dritten* Stufenbohrer **2** (Figur 8).

Die Tiefenlehre **3** gemäss Figur 5 für den Pilotbohrer **1** weist apikal die Führung **31** mit dem Durchmesser **t1,** der Länge **k1** und der zuunterst liegenden Spitze **30** auf. An die Führung **31** schliesst sich der Hals **32** mit den Tiefenmarkierungen **321** an. Dem Hals **32** folgt ein Haltebereich **33** mit dem Übergang **331** zum Kopf **332.** Der Durchmesser **t1** der Führung **31** ist gleich oder geringfügig kleiner als der Durchmesser **b1** der Pilotführung **11** des Pilotbohrers **1.** Die Länge **k1** der Führung **31** wird bevorzugt geringfügig grösser als die Länge **l1** der Pilotführung **11** des Pilotbohrers **1** gestaltet. **Der Durchmessert2** des Halses **32** der Tiefenlehre **3** wird ebenfalls gleich oder etwas kleiner als der Durchmesser **b2** des Bohrhalses **12** des Pilotbohrers **1** bemessen. Die Durchmesser und Längenverhältnisse erlauben es, dass sich die Tiefenlehre **3** zur Messung der Tiefe in ein Bohrloch problemlos einschieben lässt und durch Ermittlung der Einsinktiefe an den Tiefenmarkierungen **321** die effektive Tiefe des erzeugten Bohrlochs zuverlässig bestimmt werden kann.

Die Tiefenlehre **3** gemäss Figur 6 für den *ersten* Stufenbohrer **2** hat an ihrer Führung **31** den Durchmesser **t2',** welcher gleich oder geringfügig kleiner als der Durchmesser **b2'** an der Stufenführung **21** des *ersten* Stufenbohrers **2** ist. Die Länge **k2** der Führung **31** ist bevorzugt minimal grösser als die Länge **l2** der Stufenführung **21** dieses Stufenbohrers **2.** Der Durchmesser **t3** des Halses **32** ist ebenfalls gleich oder etwas kleiner als der Durchmesser **b3** des Bohrhalses **22** des *ersten* Stufenbohrers **2.**

Die Tiefenlehre **3** gemäss Figur 7 für den *zweiten* Stufenbohrer **2** hat an ihrer Führung **31** den Durchmesser **t3',** welcher gleich oder geringfügig kleiner als der Durchmesser **b3'** an der Stufenführung **21** des *zweiten* Stufenbohrers **2** ist. Die **Länge k3** der Führung **31** ist minimal grösser als die Länge **l3** der Stufenführung **21** dieses Stufenbohrers **2.** Der Durchmesser **t4** des Halses **32** ist ebenfalls gleich oder etwas kleiner als der Durchmesser **b4** des Bohrhalses **22** des *zweiten* Stufenbohrers **2.**

Analog ist die Tiefenlehre **3** gemäss Figur 8 für den *dritten* Stufenbohrer **2** beschaffen. Die Führung **31** hat den Durchmesser **t4',** welcher mit dem Durchmesser **b4'** an der Stufenführung **21** des *dritten* Stufenbohrers **2** korrespondiert. Die Länge **k4** der Führung **31** korrespondiert mit der Länge **l4** dieses *dritten* Stufenbohrers **2,** und der Durchmesser **t5** am Hals **32** entspricht dem Durchmesser **b5** am Bohrhals **22** des *dritten* Stufenbohrers **2.**

### Figur 9

Das Implantat **4** ist von an sich bekannter Gestalt und beginnt apikal mit der Spitze **41,** der zunächst ein Schaft **42** und als koronales Ende ein Hals **43** folgen. Von der Spitze **41** erstreckt sich eine Gewindeschneidegeometrie **44** in den Schaft **42** hinein, der mit einem Aussengewinde **421** versehen ist und den Kerndurchmesser **i3** aufweist. Stärkere Implantate **4** haben den Kerndurchmesser **i4** oder **i5** (s. Figuren 12 und 13). Von besonderer Bedeutung in Relation zur zu schaffenden Bohrung im Kieferknochen sind die Abrundung **411** an der Implantatspitze **41** und der Konus **412** mit dem Konusschneidebereich **441** sowie dem Gewindeschneidebereich **442.** Durch diese Geometrie erzielt man beim Eindrehen in eine entsprechend im Kieferknochen präparierte Bohrung mit den dabei produzierten Knochenspänen einen passgenauen Sitz für das Implantat **4,** ohne grössere Hohlräume und ohne überhöhte Knochenkompression.

### Figur 10

Nun wird das Handling mit dem Pilotbohrer **1** und der zugehörigen Tiefenmesslehre **3** erläutert. Die Mundsituation ist dabei schematisch mit dem Kieferknochen **5** dargestellt, d.h. ohne die Gingiva. Es wird angenommen, dass der Pilotbohrer **1** im Handstück einer üblichen zahnärztlichen Bohrmaschine eingesetzt ist.

### Schritt 1

Mit dem Pilotbohrer **1 -** mit seinem Durchmesser **b1** an der Pilotführung **11** und dem Durchmesser **b2** am Bohrhals **12** - wird an der vorgesehenen Position im Kieferknochen **5** die Kortikalis **51,** unter welcher die Spongiosa **52** liegt, mit der Pilotspitze **10** durchbohrt. Der Pilotbohrer **1** ist in der Bohrungsrichtung **R** orientiert. Beim Bohren verspürt der Chirurg einen erhöhten Widerstand|, sobald die Stufe **124** auf die Kortikalis aufsetzt, was er als Zeichen zur Unterbrechung des Bohrprozesses benutzt und zum zweiten Schritt überleitet.

### Schritt 2

Es wird eine optische Kontrolle der Position der in den Kieferknochen **5** ansatzweise eingebrachten Pilotbohrung **61** mit der erzeugten Pilotbohrungsspitze **610** und Pilotbohrungsführung **611,** welche die harte Kortikalis **51** durchsetzt, vorgenommen. Insbesondere wird kontrolliert, ob Bohrungsrichtung **R** der Planung entspricht. Die Pilotführung **11** mit der Pilotspitze **10** sind also dazu bestimmt, die Position der im weiteren herzustellenden Stufenbohrung **62,63,64** mit dem Einbringen eines Ansatzes einer Pilotbohrung **61** durch die Kortikalis **51** festzulegen.

### Schritt 3

Der noch still stehende Pilotbohrer **1** wird erneut in die begonnene Pilotbohrung **61** angesetzt. Innerhalb eines kegelförmigen Korrekturbereichs **K** bestimmt man die planungsgemässe Bohrungsrichtung **R** in Relation zur umgebenden Mundsituation und startet den weiteren Bohrvorgang, womit die definitive Bohrungsrichtung **R** festgelegt ist. Die stumpfen Führungsschneiden **112** ermöglichen diese Korrektur ohne Aufweitung der Pilotbohrungsführung **611.**

### Schritt 4

Die Pilotbohrung **61** in der eventuell korrigierten Bohrungsrichtung **R** wird nun in voller Bohrtiefe erzeugt. An den Tiefenmarkierungen **121** kann das Vordringen in die Spongiosa **52** kontrolliert werden.

### Schritt 5

Die zugehörige Tiefenlehre **3** mit dem Durchmesser **t2** am Hals **32** wird in die Pilotbohrung **61** eingeführt und durch Ermitteln der Einsinktiefe durch Ablesen an den Tiefenmarkierungen **321** kontrolliert man die erzeugte Pilotbohrung **61** auf ihre exakte Tiefe.

### Schritt 6

Die erzeugte Pilotbohrung 61 mit der am Bohrungsgrund liegenden Pilotbohrungsspitze **610,** der sich daran anschliessenden Pilotbohrungsführung **611** und dem nach koronal aufsteigenden Pilotbohrungshals **612**, der an der Kortikalis **51** mündet, wird optisch überprüft.

### Figur 11

Es folgt das Handling mit dem *ersten* Stufenbohrer **2** und der dazu korrespondierenden Tiefenmesslehre **3.** Es wird angenommen, dass auch der *erste* Stufenbohrer **2** und eventuell der anschliessend verwendete *zweite* und *dritte* Stufenbohrer **2** in einem zahnärztlichen Handstück eingesetzt ist.

### Schritt 6

Die erzeugte Pilotbohrung **61** wurde optisch überprüft (als Übernahme von Schritt 6 aus Figur 10).

### Schritt 7

Vom *ersten* Stufenbohrer **2** - mit dem Durchmesser **b3** am Bohrhals **22** - wird die Stufenführung **21** mit dem Bohrdurchmesser **b2'** in die Pilotbohrung **61** eingeführt und auf korrekte Ausrichtung kontrolliert.

### Schritt 8

Der Bohrprozess wird gestartet und eine *erste* Stufenbohrung **62** auf die volle Tiefe ausgebohrt. Durch die am Stufenbohrer **2** vorhandene Stufenführung **21,** deren Führungsschneiden **212** stumpf sind, und den seitlich nicht-schneidenden Bohrhals **22,** ist eine optimale Zentrierung in der Pilotbohrung **61** gewährleistet. Ein seitliches Abdriften der Bohrung wird im Prinzip ausgeschlossen. In der drei-schneidigen Ausbildung besitzt der Stufenbohrer **2** ausgezeichnete Zentriereigenschaften. Die Stufe **224** mit den Stufenschneiden **225** bewirkt, dass die Pilotbohrung **61** mit den Durchmessern **d1/d2** auf die Durchmesser **d2/d3** aufgeweitet wird.

### Schritt 9

Die zugehörige Tiefenlehre **3** mit dem Durchmesser **t3** am Hals **32** für den *ersten* Stufenbohrer **2** wird in die erzeugte *erste* Stufenbohrung **62** zur Überprüfung der erreichten Bohrtiefe eingeführt.

### Schritt 10

Optische Kontrolle der erzeugten *ersten* Stufenbohrung **62,** die sich aus der am Bohrungsgrund liegenden Stufenspitze **620,** der darauf folgenden Stufenführung **621** und dem nach koronal aufsteigenden, in der Kortikalis **51** mündenden Stufenhals **622** zusammensetzt.

### Schritt 10.1

In der nun vorhandenen *ersten* Stufenbohrung **62** kann ein Implantat **4 mit** dem kleinsten Kerndurchmesser **i3** eingesetzt werden. Hierbei schneidet sich das Implantat **4** selbst das Innengewinde im Kieferknochen **5.**

### Schritt 10.2

Das Implantat **4** mit dem kleinsten Kerndurchmesser **i3** liegt in situ in der *ersten* Stufenbohrung **62.**

### Figur 12

Ist beabsichtigt, ein Implantat **4** mit einem grösseren Kerndurchmesser als **i3** einzusetzen, folgt das Prozedere mit dem *zweiten* Stufenbohrer **2** und der zugehörigen Tiefenmesslehre **3.**

### Schritt 10

Die erzeugte *erste* Stufenbohrung **62** wurde optisch überprüft (als Übernahme von Schritt 10 aus Figur 11).

### Schritt 11

Vom *zweiten* Stufenbohrer **2** - mit dem Durchmesser **b4** am Bohrhals **22** - wird die Stufenführung **21** mit dem Bohrdurchmesser **b3'** in die vorhandene *erste* Stufenbohrung **62** eingeführt und auf korrekte Ausrichtung kontrolliert.

### Schritt 12

Der Bohrprozess wurde gestartet und eine *zweite* Stufenbohrung **63** auf die volle Tiefe ausgebohrt. Die Stufe **224** mit den Stufenschneiden **225** bewirkt, dass die *erste* Stufenbohrung **62** mit den Durchmessern **d2/d3** in der entstandenen *zweiten* Stufenbohrung **63** auf die Durchmesser **d3/d4** aufgeweitet wird.

### Schritt 13

Die entsprechende Tiefenlehre **3** mit dem Durchmesser **t4** am Hals **32** für den *zweiten* Stufenbohrer **2** wird in die erzeugte *zweite* Stufenbohrung **63** zur Kontrolle der erreichten Bohrtiefe eingeführt.

### Schritt 14

Optische Kontrolle der erzeugten *zweiten* Stufenbohrung **63,** die sich in Analogie zur ersten Stufenbohrung **62** aus der am Bohrungsgrund liegenden Stufenspitze **630,** der folgenden Stufenführung **631** und dem nach koronal aufsteigenden Stufenhals **632** zusammensetzt.

### Schritt 14.1

In der nun vorhandenen *zweiten* Stufenbohrung **63** lässt sich ein Implantat **4** mit dem mittleren **Kerndurchmesser i4** einsetzen.

### Schritt 14.2

Das Implantat **4** mit dem mittleren Kerndurchmesser **i4** liegt in situ in der *zwei*/-*ten* Stufen bohrung **62.**

### Figur 13

Will man ein Implantat **4** mit dem grössten Kerndurchmesser als **i5** einsetzen, folgt das Prozedere mit dem *dritten* Stufenbohrer **2** und der zugehörigen Tiefenmesslehre **3**.

### Schritt 14

Die erzeugte *zweite* Stufenbohrung **63** wurde optisch überprüft (als Übernahme von Schritt 14 aus Figur 12).

### Schritt 15

Vom *dritten* Stufenbohrer **2** - mit dem Durchmesser **b5** am Bohrhals **22 -** wird die Stufenführung **21** mit dem Bohrdurchmesser **b4'** in die vorhandene *zweite* Stufenbohrung **63** eingeführt und wiederum auf korrekte Bohrungsrichtung **R** kontrolliert.

### Schritt 16

Der Bohrprozess wurde ausgeführt und eine *dritte* Stufenbohrung **64** auf die volle Tiefe gebohrt. Die Stufe **224** mit den Stufenschneide **225** bewirkte, dass die *zweite* Stufenbohrung **63** mit den Durchmessern **d3/d4** in der entstandenen *dritten* Stufenbohrung **64** auf die Durchmesser **d4/d5** aufgeweitet wird.

### Schritt 17

Die korrespondierende Tiefenlehre **3** mit dem Durchmesser **t5** am Hals **32** für den *dritten* Stufenbohrer **2** wird in die erzeugte *dritte* Stufenbohrung **64** zur Kontrolle der exakten Bohrtiefe eingeführt.

### Schritt 18

Optische Kontrolle der erzeugten *dritten* Stufenbohrung **64,** die sich analog zu den vorherigen Stufenbohrungen **62,63** aus der Stufenspitze **640, der** Stufenführung **641** und dem Stufenhals **642** zusammensetzt.

### Schritt 18.1

In der nun vorhandenen *dritten* Stufenbohrung **62** wird ein Implantat **4** mit dem grössten Kerndurchmesser **i5** eingesetzt.

### Schritt 18.2

Das Implantat **4** mit dem grössten Kerndurchmesser **i5** liegt in situ in der *dritten* Stufenbohrung **64.**

### Figuren 14A und 14B

In dieser schematischen Darstellung liegen alle Bohrquerschnitte **61,62,63,64** übereinander, wie sie im Kieferknochen **5** vor dem Einsetzen eines Implantats **4** mit dem grössten Durchmesser **i5 in** der Abfolge der zuvor beschriebenen Arbeitszyklen angebracht werden. Es ist ersichtlich, dass um die Pilotbohrung **61 -** mit der Pilotbohrungsspitze **610,** der Pilotbohrungsführung **611** und dem Pilotbohrungshals **612** - alle weiteren danach angebrachten Stufenbohrungen **62,63,64** - mit den entsprechenden Stufenspitzen **620,630,640,** den zugehörigen Stufenführungen **621,631,641** und Stufenhälsen **622,632,642** - zentriert sind und die gleiche Bohrtiefe aufweisen.

Der Durchmesser **b2** am Bohrhals **12** des Pilotbohrers **1** ist zumindest nahezu identisch zum Durchmesser **b2'** an der Stufenführung **21** des *ersten* Stufenbohrers **2.** Somit ergeben sich auch bei den resultierenden Bohrungen zumindest nahezu identische Durchmesser, nämlich beim Pilotbohrungshals **612** und der *ersten* Stufenführung **621,** welche beide mit **d2** bezeichnet sind. Der Bohrungsdurchmesser **d1** wurde von der Pilotführung **11** des Pilotbohrers **1** mit dem Bohrungsdurchmesser **b1** erzeugt. Die Bohrungsdurchmesser **d3** bzw. **d4** stammen von den zumindest nahezu identischen Durchmessern **b3** am Bohrhals **12** des *ersten* Stufenbohrers **2** und dem Durchmesser **b3'** an der Stufenführung **21** des *zweiten* Stufenbohrers **2** bzw. von den Durchmessern **b4** am Bohrhals **12** des *zweiten* Stufenbohrers **2** und dem Durchmesser **b4'** an der Stufenführung **21** des *dritten* Stufenbohrers **2.**

Es kann vorteilhaft sein, den Bohrerdurchmesser **b2',b3',b4'** am jeweiligen Stufenbohrer **2** geringfügig - z.B. 1/100mm bis 1/10mm - kleiner zu bemessen als den Bohrerdurchmesser **b2,b3,b4** am Bohrhals **12,22** des vorangehend zu benutzenden Werkzeugs, was der Pilotbohrer **1,** der erste Stufenbohrer **2** bzw. der *zweite* Stufenbohrer **2** ist. Damit wird das Einführen und Eindringen der aussen nicht-schneidenden Stufenführung **21** mit den stumpfen Führungsschneiden **212** in den Pilothals **612** der Pilotbohrung **61** bzw. in den Stufenhals **622,632** der *ersten* bzw. *zweiten* Stufenbohrung **62,63** erleichtert. Die konkrete Durchmesserverminderung von **b2',b3',b4'** gegenüber den Bohrerdurchmessern **b2,b3,b4** am Bohrhals **12,22** wird der Fachmann **u.a.** nach den Schneideigeschaften des Bohrhalses **12,22** mit der Fase **123,223** sowie der Stufenführung **21** mit dem Spitzenwinkel β und den Führungsschneiden **212** bestimmen.

### Figur 14C

Prinzipiell ist dargestellt, wie die Spitze **41** des Implantats **4** mit dem grössten Durchmesser i5 im Querschnitt der *dritten* Stufenbohrung **64** mit Durchmesser **d5** liegt. Die Abrundung **411** von der Implantatspitze **41** passt sich gut in die Stufenspitze **640** der *dritten* Stufenbohrung **64** ein. Der Querschnitt der Stufenführung **641** der hiesigen Stufenbohrung **64** ist minimal kleiner als der Querschnitt des Konus **412** der Implantatspitze **41.** Um das Implantat **4** jedoch in die gezeigte Position einzubringen, weist dieses eine Schneidengeometrie **44** mit einem Konusschneidebereich **441** auf. Dadurch werden im Bereich des Konus **412** Partikel vom Knochen **5** abgeschnitten und der Bohrungsquerschnitt entsprechend erweitert. Diese abgetrennten Knochenpartikel werden in die Schneide **44** oder benachbarte Gebiete transportiert, wo die *dritte* Stufenbohrung **64** im Querschnitt minimal grösser ist als das hier verwendete Implantat **4** mit dem grössten Kerndurchmesser **i5.** Damit wird eine überhöhte Knochenkompression vermieden und eine optimale Primärstabilität der Spitze **41** des Implantats **4** direkt nach Implantation erreicht. Ein analoger Vorgang mit abgeschnittenen Knochenpartikeln findet im Bereich des Aussengewindes **421** des Implantats 4 und dem Stufenhals **642** der *dritten* Stufenbohrung **64** statt. Der Stufenhals **642** mit dem Durchmesser d5 ist geringfügig grösser als der Kerndurchmesser **i5** des Implantats **4,** so dass sich dort Raum für antransportierte Knochenpartikel bietet, welche durch den Gewindeschneidebereich **442** abgeschnitten wurden.

## Patentansprüche

1. Pilotbohrer **(1)** für die Vorbereitung einer in einen Kieferknochen (5) einzubringenden sacklochförmigen Stufenbohrung **(62,63,64)** zur Aufnahme eines Dentalimplantats **(4),** mit:
a) einer Pilotspitze **(10),** die am apikalen Ende des Pilotbohrers **(1)** angeordnet ist und Spitzenschneiden **(101)** hat;
b) einer Pilotführung **(11),** die sich von der Pilotspitze **(10)** in Richtung des koronalen Endes des Pilotbohrers **(1)** erstreckt;
c) einem Bohrhals **(12),** der oberhalb der Pilotführung **(11)** liegt und einen grösseren Bohrerdurchmesser **(b2)** aufweist als der Bohrerdurchmesser **(b1)** der Pilotführung **(11);**
d) einem oberhalb des Bohrhalses **(12)** liegenden Bohrschaft **(13),** an den sich als koronales Ende eine Kupplung **(14)** anschliessen kann;
e) zumindest einer seitlich an der Pilotführung **(11)** liegenden Führungsschneide **(112);**
f) einer Stufe **(124),** als Übergang von der Pilotführung **(11) zum** Bohrhals **(12);**
g) zumindest einer Stufenschneide **(125)** an der Stufe **(124);** und
h) zumindest einer Spiralnut **(122)** und einer daran angrenzenden Fase **(123), dadurch gekennzeichnet, dass**
i) die Spitzenschneiden **(101)** an der Pilotspitze **(10)** scharf ausgebildet und zentrums-schneidend sind;
j) sich Anschliffe **(111)** von den Spitzenschneiden **(101)** aufwärts der Pilotführung **(11)** erstrecken;
k) die Stufenschneiden **(125)** an der Stufe **(124)** schneidend ausgebildet sind; und
l) die Führungsschneiden **(112)** stumpf, nicht-schneidend ausgebildet sind.

2. Pilotbohrer **(1)** nach Anspruch 1, **dadurch gekennzeichnet, dass**
a) der Bohrhals **(12)** mit der Fase **(123)** schwach schneidend ausgebildet ist; und
b) die Pilotführung **(11)** eine Länge (**l1**) im Bereich von 1.0mm bis 4.0mm aufweist.

3. Pilotbohrer **(1)** nach Anspruch 1 oder 2, **dadurch gekennzeichnet, dass**
a) der Pilotbohrer **(1)** zweischneidig ausgebildet ist und somit jeweils zwei Spitzenschneiden **(101),** Anschliffe **(111),** Führungsschneiden (**112**), Spiralnuten **(122),** Fasen **(123)** und Stufenschneiden **(125)** aufweist;
b) der Bohrhals **(12)** mindestens die Länge der Einsetztiefe des zu applizierenden Implantats **(4)** hat;
c) die Pilotführung **(11)** die Länge (**l1**) von 3.0mm hat;
d) die Pilotführung **(11)** den Durchmesser **(b1)** im Bereich von 1.5mm aufweist und der Bohrhals **(12)** den Durchmesser **(b2)** im Bereich von 2.0mm aufweist;
e) der zwischen den Spitzenschneiden **(101)** liegende Spitzenwinkel (α) kleiner als 90° ist, vorzugsweise im Bereich von 80° liegt;
f) die Spiralnuten **(122)** sich durchgängig vom koronalen Ende des Bohrhalses (**12**) bis in die Pilotspitze **(10)** erstrecken, wobei an der Pilotführung (11) die Spiralnuten **(122)** durch den geringeren Durchmesser **(b1)** nur mehr einen Anteil ihres vollen Querschnitts, wie er am Bohrhals (12) vorhanden ist, aufweisen;
g) am Bohrhals **(12)** mehrere sichtbare Tiefenmarkierungen **(121)** in gleichen oder ungleichen Abständen angebracht sind; und
h) die sich an den Bohrschaft **(13)** anschliessende Kupplung **(14)** eine standardisierte Dentalkupplung ist.

4. Pilotbohrer **(1)** nach einem der Ansprüche 1 bis 3, **dadurch gekennzeichnet, dass**
a) die Pilotführung **(11)** mit der Pilotspitze **(10)** dazu bestimmt sind, die Position der zu erzeugenden Stufenbohrung **(62,63,64)** mit dem Einbringen eines Ansatzes einer Pilotbohrung **(61)** durch die Kortikalis **(51)** des Kieferknochens **(5)** festzulegen, wobei der Ansatz aus einer Pilotbohrungsführung **(611)** und einer Pilotbohrungsspitze **(610)** besteht;
b) die Stufe **(124)** dazu bestimmt ist, nach durchdrungener Kortikalis **(51)** mit Fertigstellung der Pilotbohrungsführung **(611)** und -spitze **(610)** einen spürbar erhöhten Bohrwiderstand zu generieren und bei diesem Anzeichen die angesetzte Bohrungsrichtung **(R)** zu überprüfen;
c) die stumpfen Führungsschneiden **(112)** ermöglichen, ohne Aufweitung der Pilotbohrungsführung **(611),** die Bohrungsrichtung **(R)** innerhalb eines kegelförmigen Korrekturbereichs **(K)** zu korrigieren; und
d) der Bohrhals **(12)** mit seiner Dimensionierung dazu bestimmt ist, die Pilotbohrung **(61)** in der endgültigen Tiefe zu erstellen.

5. Stufenbohrer **(2)** für die Vergrösserung einer in einem Kieferknochen **(5)** vorhandenen sacklochförmigen Pilotbohrung **(61)** zu einer Stufenbohrung **(62)** bzw. für die weitere Vergrösserung einer vorhandenen Stufenbohrung **(62,63)** zu einer nochmals vergrösserten Stufenbohrung (**63,64**) als Aufnahme für ein Dentalimplantat **(4),** mit:
a) einer Stufenspitze **(20),** die am apikalen Ende des Stufenbohrers **(2)** angeordnet ist und Spitzenschneiden **(201)** hat;
b) einer Stufenführung **(21),** die sich von der Stufenspitze **(20)** in Richtung des koronalen Endes des Stufenbohrers **(2)** erstreckt;
c) einem Bohrhals **(22),** der oberhalb der Stufenführung **(21)** liegt und einen grösseren Bohrerdurchmesser **(b3,b4,b5)** aufweist als der Bohrerdurchmesser **(b2',b3',b4')** der Stufenführung **(21);**
d) einem oberhalb des Bohrhalses **(22)** liegenden Bohrschaft **(23),** an den sich als koronales Ende eine Kupplung **(24)** anschliessen kann;
e) zumindest einer seitlich an der Stufenführung **(21)** liegenden Führungsschneide **(212);**
f) einer Stufe **(224),** als Übergang von der Stufenführung **(21)** zum Bohrhals **(22);**
g) zumindest einer Stufenschneide **(225)** an der Stufe **(224);** und
h) zumindest einer Spiralnut **(222)** und einer daran angrenzenden Fase **(223), dadurch gekennzeichnet, dass**
i) die Spitzenschneiden **(201)** an der Stufenspitze **(20)** scharf ausgebildet sind;
j) sich Anschliffe **(211)** von den Spitzenschneiden **(201)** aufwärts der Stufenführung **(21)** erstrecken;
k) die Stufenschneiden **(225)** an der Stufe **(224)** schneidend ausgebildet sind; und
l) die Führungsschneiden **(212)** stumpf, nicht-schneidend ausgebildet sind.

6. Stufenbohrer **(2)** nach Anspruch 5, **dadurch gekennzeichnet, dass**
a) der Bohrhals **(22)** mit der Fase **(223)** schwach schneidend ausgebildet ist; und
b) die Stufenführung **(21)** eine Länge **(l2,l3,l4)** im Bereich von 2.0mm aufweist.

7. Stufenbohrer **(2)** nach Anspruch 5 oder 6, **dadurch gekennzeichnet, dass**
a) der Stufenbohrer **(2)** dreischneidig ausgebildet ist und somit jeweils drei Spitzenschneiden **(201),** Anschliffe **(211),** Führungsschneiden (**212**), Spiralnuten **(222),** Fasen (**223**) und Stufenschneiden **(225)** aufweist;
b) der Bohrhals **(22)** mindestens die Länge der Einsetztiefe des zu applizierenden Implantats **(4)** hat;
c) die Stufenführung **(21)** den Durchmesser **(b2',b3',b4')** im Bereich von 2.0mm, 2.8mm, 3.5mm aufweist und der Bohrhals (**22**) den Durchmesser **(b3,b4,b5)** im Bereich von 2.8mm, 3.5mm, 4.3mm aufweist;
d) der zwischen den Spitzenschneiden **(201)** liegende Spitzenwinkel (β) grösser als 90° ist, vorzugsweise im Bereich von 120° liegt;
e) die Spiralnuten **(222)** sich durchgängig vom koronalen Ende des Bohrhalses **(22)** bis in die Stufenspitze **(20)** erstrecken, wobei an der Stufenführung **(21) die** Spiralnuten **(222)** durch den geringeren Durchmesser **(b2',b3',b4')** nur mehr einen Anteil ihres vollen Querschnitts, wie er am Bohrhals **(22)** vorhanden ist, aufweisen;
f) am Bohrhals **(22)** mehrere sichtbare Tiefenmarkierungen **(221)** in gleichen oder ungleichen Abständen angebracht sind; und
g) die sich an den Bohrschaft **(23)** anschliessende Kupplung **(24)** eine standardisierte Dentalkupplung ist.

8. Stufenbohrer **(2)** nach einem der Ansprüche 5 bis 7, **dadurch gekennzeichnet, dass**
a) die Stufenführung **(21)** mit der Stufenspitze **(10)** und den stumpfen Führungsschneiden **(212)** dazu bestimmt ist, den Stufenbohrer **(2)** beim Ansetzen in die Pilotbohrung **(61)** bzw. Stufenbohrung **(62,63)** zu zentrieren und beim Vortrieb entlang der Pilotbohrung **(61)** bzw. der Stufenbohrung (**62,63**) zentriert zu führen; und
b) die Stufe **(224)** mit den Stufenschneiden (**225**) dazu bestimmt ist, die Pilot-bohrung **(61)** mit den Durchmessern (**d1/d2**) auf die Durchmesser **(d2/d3)** aufzuweiten bzw. die Stufenbohrung **(62,63)** mit den Durchmessern (**d2/d3,d3/d4**) auf die Durchmesser (**d3/d4,d4/d5**) der Stufenbohrung **(63,64)** aufzuweiten.

9. Bohrerset bestehend aus:
a) einem Pilotbohrer **(1)** zur Erstellung einer sacklochförmigen Pilotbohrung **(61)** als Vorbereitung für eine in einen Kieferknochen **(5)** einzubringenden sacklochförmigen Stufenbohrung **(62,63,64)** zur Aufnahme eines Dentalimplantats **(4);**
b) einem *ersten* Stufenbohrer **(2)** für die Vergrösserung der vorhandenen Pilotbohrung **(61)** zu einer Stufenbohrung **(62);**
c) einem *zweiten* Stufenbohrer **(2)** für die zweite Vergrösserung einer vorhandenen Stufenbohrung **(62)** zu einer nochmals vergrösserten Stufenbohrung (**63**); und
d) einem *dritten* Stufenbohrer **(2)** für die dritte Vergrösserung der bereits zweifach vergrösserten Stufenbohrung **(63)** zu einer letztmalig vergrösserten Stufenbohrung **(64);** wobei
e) der Pilotbohrer **(1)** aufweist:
ea) eine Pilotspitze **(10),** die am apikalen Ende des Pilotbohrers **(1)** angeordnet ist und Spitzenschneiden **(101)** hat;
eb) eine Pilotführung **(11),** die sich von der Pilotspitze **(10)** in Richtung des koronalen Endes des Pilotbohrers **(1)** erstreckt;
ec) einen Bohrhals **(12),** der oberhalb der Pilotführung **(11)** liegt und einen grösseren Bohrerdurchmesser (**b2**) aufweist als der Bohrerdurchmesser **(b1)** der Pilotführung **(11);**
ed) einen oberhalb des Bohrhalses **(12)** liegenden Bohrschaft **(13),** an den sich als koronales Ende eine Kupplung **(14)** anschliessen kann;
ee) zumindest eine seitlich an der Pilotführung **(11)** liegende Führungsschneide **(112);**
ef) eine Stufe **(124),** als Übergang von der Pilotführung **(11)** zum Bohrhals (**12**);
eg) zumindest eine Stufenschneide **(125)** an der Stufe **(124);** und
eh) zumindest eine Spiralnut **(122)** und eine daran angrenzende Fase **(123);** und
f) der Stufenbohrer **(2)** aufweist:
fa) eine Stufenspitze **(20),** die am apikalen Ende des Stufenbohrers (**2**) angeordnet ist und Spitzenschneiden **(201)** hat;
fb) eine Stufenführung **(21),** die sich von der Stufenspitze **(20)** in Richtung des koronalen Endes des Stufenbohrers **(2)** erstreckt;
fc) einen Bohrhals **(22),** der oberhalb der Stufenführung **(21)** liegt und einen grösseren Bohrerdurchmesser **(b3,b4,b5)** aufweist als der Bohrerdurchmesser (**b2',b3',b4'**) der Stufenführung **(21);**
fd) einen oberhalb des Bohrhalses **(22)** liegenden Bohrschaft **(23),** an den sich als koronales Ende eine Kupplung **(24)** anschliessen kann;
fe) zumindest eine seitlich an der Stufenführung **(21)** liegende Führungsschneide **(212);**
ff) eine Stufe **(224),** als Übergang von der Stufenführung **(21)** zum Bohrhals **(22);**
fg) zumindest eine Stufenschneide **(225)** an der Stufe **(224);** und
fh) zumindest eine Spiralnut **(222)** und eine daran angrenzende Fase **(223), dadurch gekennzeichnet, dass**
g) am Pilotbohrer **(1):**
ga) die Spitzenschneiden **(101)** an der Pilotspitze **(10)** scharf ausgebildet und zentrums-schneidend sind;
gb) sich Anschliffe **(111)** von den Spitzenschneiden **(101)** aufwärts der Pilotfüh-rung **(11)** erstrecken;
gc) die Stufenschneiden **(125)** an der Stufe **(124)** schneidend ausgebildet sind; und
gd) die Führungsschneiden **(112)** stumpf, nicht-schneidend ausgebildet sind;
h) am Stufenbohrer **(2):**
ha) die Spitzenschneiden **(201)** an der Stufenspitze **(20)** scharf ausgebildet sind;
hb) sich Anschliffe **(211)** von den Spitzenschneiden **(201)** aufwärts der Stufenführung **(21)** erstrecken;
hc) die Stufenschneiden **(225)** an der Stufe **(224)** schneidend ausgebildet sind; und
hd) die Führungsschneiden **(212)** stumpf, nicht-schneidend ausgebildet sind;
i) der Durchmesser **(b2')** des *ersten* Stufenbohrers **(2)** an der Stufenführung **(21)** dem Durchmesser **(b2)** am Bohrhals **(12)** des Pilotbohrers **(1)** entspricht; und
j) der Durchmesser (**b3',b4'**) des *zweiten* bzw. *dritten* Stufenbohrer **(2)** an der Stufenführung **(21)** dem Durchmesser (**b3,b4**) am Bohrhals **(22)** des vorangehenden *ersten* bzw. *zweiten* Stufenbohrers **(2)** entspricht.

10. Bohrerset nach Anspruch 9, **dadurch gekennzeichnet, dass** vom Pilotbohrer **(1)** der Bohrhals **(12)** mit der Fase **(123)** schwach schneidend ausgebildet ist und dessen Pilotführung **(11)** eine Länge (**l1**) im Bereich von 1.0mm bis 4.0mm aufweist.

11. Bohrerset nach Anspruch 9 oder 10, **dadurch gekennzeichnet, dass**
a) der Pilotbohrer **(1)** zweischneidig ausgebildet ist und somit jeweils zwei Spitzenschneiden **(101),** Anschliffe **(111),** Führungsschneiden **(112),** Spiralnuten **(122),** Fasen **(123)** und Stufenschneiden **(125)** aufweist;
b) der Bohrhals **(12)** mindestens die Länge der Einsetztiefe des zu applizierenden Implantats **(4)** hat;
c) die Pilotführung **(11)** die Länge **(l1)** von 3.0mm hat;
d) die Pilotführung **(11)** den Durchmesser **(b1)** im Bereich von 1.5mm aufweist und der Bohrhals **(12)** den Durchmesser **(b2)** im Bereich von 2.0mm aufweist;
e) der zwischen den Spitzenschneiden **(101)** liegende Spitzenwinkel (α) kleiner als 90° ist, vorzugsweise im Bereich von 80° liegt;
f) die Spiralnuten **(122)** sich durchgängig vom koronalen Ende des Bohrhalses **(12)** bis in die Pilotspitze **(10)** erstrecken, wobei an der Pilotführung **(11)** die Spiralnuten **(122)** durch den geringeren Durchmesser **(b1)** nur mehr einen Anteil ihres vollen Querschnitts, wie er am Bohrhals (**12**) vorhanden ist, aufweisen;
g) am Bohrhals **(12)** mehrere sichtbare Tiefenmarkierungen **(121)** in gleichen oder ungleichen Abständen angebracht sind; und
h) die sich an den Bohrschaft **(13)** anschliessende Kupplung (**14**) eine standardisierte Dentalkupplung ist.

12. Bohrerset nach einem der Ansprüche 9 bis 11, **dadurch gekennzeichnet, dass**
a) die Pilotführung **(11)** mit der Pilotspitze **(10)** dazu bestimmt sind, die Position der zu erzeugenden Stufenbohrung **(62,63,64)** mit dem Einbringen eines Ansatzes einer Pilotbohrung **(61)** durch die Kortikalis **(51)** des Kieferknochens **(5)** festzulegen, wobei der Ansatz aus einer Pilotbohrungsführung **(611)** und einer Pilotbohrungsspitze **(610)** besteht;
b) die Stufe **(124)** dazu bestimmt ist, nach durchdrungener Kortikalis **(51)** mit Fertigstellung der Pilotbohrungsführung **(611)** und -spitze **(610)** einen spürbar erhöhten Bohrwiderstand zu generieren und bei diesem Anzeichen die angesetzte Bohrungsrichtung **(R)** zu überprüfen;
c) die stumpfen Führungsschneiden **(112)** ermöglichen, ohne Aufweitung der Pilotbohrungsführung **(611),** die Bohrungsrichtung **(R)** innerhalb eines kegelförmigen Korrekturbereichs **(K)** zu korrigieren; und
d) der Bohrhals **(12)** mit seiner Dimensionierung dazu bestimmt ist, die Pilotbohrung **(61)** in der endgültigen Tiefe zu erstellen.

13. Bohrerset nach Anspruch 9, **dadurch gekennzeichnet, dass** vom Stufenbohrer **(2)** der Bohrhals **(22)** mit der Fase (**223**) schwach schneidend ausgebildet ist und dessen Stufenführung (**21**) eine Länge (**l2,l3,l4**) im Bereich von 2.0mm aufweist.

14. Bohrerset nach Anspruch 9 oder 13, **dadurch gekennzeichnet, dass**
a) der Stufenbohrer **(2)** dreischneidig ausgebildet ist und somit jeweils drei Spitzenschneiden **(201),** Anschliffe **(211),** Führungsschneiden (**212**), Spiralnuten **(222),** Fasen **(223)** und Stufenschneiden **(225)** aufweist;
b) der Bohrhals **(22)** mindestens die Länge der Einsetztiefe des zu applizierenden Implantats **(4)** hat;
c) die Stufenführung **(21)** den Durchmesser **(b2',b3',b4')** im Bereich von 2.0mm, 2.8mm, 3.5mm aufweist und der Bohrhals **(22)** den Durchmesser (**b3,b4,b5**) im Bereich von 2.8mm, 3.5mm, 4.3mm aufweist;
d) der zwischen den Spitzenschneiden **(201)** liegende Spitzenwinkel (β) grösser als 90° ist, vorzugsweise im Bereich von 120° liegt;
e) die Spiralnuten **(222)** sich durchgängig vom koronalen Ende des Böhrhalses (**22**) bis in die Stufenspitze **(20)** erstrecken, wobei an der Stufenführung **(21)** die Spiralnuten **(222)** durch den geringeren Durchmesser **(b2',b3',b4')** nur mehr einen Anteil ihres vollen Querschnitts, wie er am Bohrhals **(22)** vorhanden ist, aufweisen;
f) am Bohrhals **(22)** mehrere sichtbare Tiefenmarkierungen (**221**) in gleichen oder ungleichen Abständen angebracht sind; und
g) die sich an den Bohrschaft **(23)** anschliessende Kupplung **(24)** eine standardisierte Dentalkupplung ist.

15. Bohrerset nach einem der Ansprüche 9, 13 oder 14, **dadurch gekennzeichnet, dass**
a) die Stufenführung **(21)** mit der Stufenspitze **(10)** und den stumpfen Führungsschneiden **(212)** dazu bestimmt ist, den Stufenbohrer (2) beim Ansetzen in die Pilotbohrung **(61)** bzw. Stufenbohrung (**62,63**) zu zentrieren und beim Vortrieb entlang der Pilotbohrung **(61)** bzw. der Stufenbohrung **(62,63)** zentriert zu führen; und
b) die Stufe **(224)** mit den Stufenschneiden **(225)** dazu bestimmt ist, die Pilotbohrung **(61)** mit den Durchmessern (**d1/d2**) auf die Durchmesser (**d2/d3**) aufzuweiten bzw. die Stufenbohrung **(62,63)** mit den Durchmessern **(d2/d3,d3/d4)** auf die Durchmesser **(d3/d4,d4/d5)** der Stufenbohrung **(63,64)** aufzuweiten.

## Claims

1. A pilot drill **(1)** for the preparation of a step bore **(62,63,64)** in the form of a blind hole, to be introduced into a jawbone **(5),** for receiving a dental implant **(4),** with:
a) a pilot tip **(10),** which is arranged at the apical end of the pilot drill **(1)** and has tip cutting edges **(101);**
b) a pilot guide **(11),** which extends from the pilot tip **(10)** in the direction of the coronal end of the pilot drill **(1);**
c) a drill neck **(12),** which lies above the pilot guide **(11)** and has a larger drill diameter **(b2)** than the drill diameter **(b1)** of the pilot guide **(11);**
d) a drill stem **(13),** which lies above the drill neck **(12)** and may be adjoined by a coupling **(14)** as the coronal end;
e) at least one guide cutting edge **(112)** lying to the side of the pilot guide **(11);**
f) a step **(124),** as a transition from the pilot guide **(11)** to the drill neck **(12);**
g) at least one step cutting edge **(125)** at the step **(124);** and
h) at least one spiral groove **(122)** and an adjacent bevel **(123),**
**characterized in that**
i) the tip cutting edges **(101)** at the pilot tip **(10)** are sharply formed and center-cutting;
j) chamfers **(111)** extend from the tip cutting edges **(101)** upward of the pilot guide **(11);**
k) the step cutting edges **(125)** at the step **(124)** are formed in a cutting manner; and
l) the guide cutting edges **(112)** are formed in a blunt, non-cutting manner.

2. The pilot drill **(1)** as claimed in claim 1, **characterized in that**
a) the drill neck **(12)** with the bevel **(123)** is formed in a weakly cutting manner; and
b) the pilot guide **(11)** has a length **(l1)** in the range from 1.0 mm to 4.0 mm.

3. The pilot drill **(1)** as claimed in claim 1 or 2, **characterized in that**
a) the pilot drill **(1)** is formed with two cutting edges and consequently has two tip cutting edges **(101),** two chamfers **(111),** two guide cutting edges **(112),** two spiral grooves **(122),** two bevels **(123)** and two step cutting edges **(125);**
b) the drill neck **(12)** has a length at least equal to the depth of insertion of the implant **(4)** to be applied;
c) the pilot guide **(11)** has a length **(l1)** of 3.0 mm;
d) the pilot guide **(11)** has a diameter **(b1)** in the region of 1.5 mm and the drill neck **(12)** has a diameter **(b2)** in the region of 2.0 mm;
e) the tip angle (α) lying between the tip cutting edges **(101)** is less than 90°, preferably lies in the region of 80°;
f) the spiral grooves **(122)** extent continuously from the coronal end of the drill neck **(12)** into the pilot tip **(10),** the spiral grooves **(122)** having at the pilot guide **(11)** only a fraction of their full cross section, as present at the drill neck (**12**), as a result of the smaller diameter **(b1);**
g) a number of visible depth markings **(121)** are provided at equal or unequal intervals on the drill neck **(12);** and
h) the coupling **(14)** adjoining the drill stem **(13)** is a standardized dental coupling.

4. The pilot drill **(1)** as claimed in one of claims 1 to 3, **characterized in that**
a) the pilot guide **(11)** with the pilot tip **(10)** are intended to fix the position of the step bore **(62,63,64)** that is to be produced, by introducing a start of a pilot bore **(61)** through the cortical bone **(51)** of the jawbone **(5),** the start comprising the pilot bore guide **(611)** and a pilot bore tip **(610);**
b) the step **(124)** is intended for generating a noticeably increased drilling resistance once the cortical bone **(51)** is penetrated, with completion of the pilot bore guide **(611)** and tip **(610),** and using this indication to check the drilling direction **(R)** that has been set up;
c) the blunt guide cutting edges **(112)** make it possible to correct the drilling direction **(R)** within a conical range of correction **(K)** without widening the pilot bore guide **(611);** and
d) the drill neck **(12)** with its dimensioning is intended to create the pilot bore **(61)** with the final depth.

5. A step drill **(2)** for the enlargement of a pilot bore **(61)** in the form of a blind hole present in a jawbone **(5)** into a step bore **(62)** or for the further enlargement of an existing step bore **(62,63)** into a further enlarged step bore **(63,64)** as a receptacle for a dental implant **(4),** with
a) a step tip **(20),** which is arranged at the apical end of the step drill **(2)** and has tip cutting edges **(201);**
b) a step guide **(21),** which extends from the step tip **(20)** in the direction of a coronal end of the step drill **(2);**
c) a drill neck **(22),** which lies above the step guide **(21)** and has a larger drill diameter **(b3,b4,b5)** than the drill diameter **(b2',b3',b4')** of the step guide (**21**);
d) a drill stem **(23),** which lies above the drill neck **(22)** and may be adjoined by a coupling **(24)** as the coronal end;
e) at least one guide cutting edge **(212)** lying to the side of the step guide (**21**);
f) a step **(224),** as a transition from the step guide **(21)** to the drill neck **(22);**
g) at least one step cutting edge **(225)** at the step **(224);** and
h) at least one spiral groove **(222)** and an adjacent bevel **(223), characterized in that**
i) the tip cutting edges **(201)** at the step tip **(20)** are sharply formed;
j) chamfers **(211)** extend from the tip cutting edges **(201)** upward of the step guide **(21);**
k) the step cutting edges **(225)** at the step **(224)** are formed in a cutting manner; and
l) the guide cutting edges **(212)** are formed in a blunt, non-cutting manner.

6. The step drill **(2)** as claimed in claim 5, **characterized in that**
a) the drill neck **(22)** with the bevel **(223)** is formed in a weakly cutting manner; and
b) the step guide **(21)** has a length (**l2,l3,l4**) in the region of 2.0 mm.

7. The step drill **(2)** as claimed in claim 5 or 6, **characterized in that**
a) the step drill **(2)** is formed with three cutting edges and consequently has three tip cutting edges **(201),** three chamfers **(211),** three guide cutting edges **(212),** three spiral grooves **(222),** three bevels **(223)** and three step cutting edges **(225);**
b) the drill neck **(22)** has a length at least equal to the depth of insertion of the implant **(4)** to be applied;
c) the step guide **(21)** has a diameter **(b2',b3',b4')** in the region of 2.0 mm, 2.8 mm and 3.5 mm, respectively, and the drill neck **(22)** has a diameter **(b3,b4,b5)** in the region of 2.8 mm, 3.5 mm and 4.3 mm, respectively;
d) the tip angle (β) lying between the tip cutting edges **(201)** is more than 90°, preferably lies in the region of 120°;
e) the spiral grooves **(222)** extend continuously from the coronal end of the drill neck **(22)** into the step tip **(20),** the spiral grooves **(222)** having at the step guide **(21)** only a fraction of their full cross section, as present at the drill neck **(22),** as a result of the smaller diameter (**b2',b3',b4'**);
f) a number of visible depth markings **(221)** being provided at equal or unequal intervals on the drill neck **(22);** and
g) the coupling **(24)** adjoining the drill stem **(23)** being a standardized dental coupling.

8. The step drill **(2)** as claimed in one of claims 5 to 7, **characterized in that**
a) the step guide **(21)** with the step tip **(10)** and the blunt guide cutting edges **(212)** is intended for centering the step drill **(2)** when setting it up in the pilot bore **(61)** or step bore (**62,63**) and guiding it in a centered manner when advancing along the pilot bore **(61)** or the step bore **(62,63);** and
b) the step **(224)** with the step cutting edges **(225)** is intended for widening the pilot bore **(61)** with the diameters (**d1/d2**) to the diameters **(d2/d3)** or for widening the step bore **(62,63)** with the diameters **(d2/d3, d3/d4)** to the diameters **(d3/d4,d4/d5)** of the step bore **(63,64).**

9. A drill set comprising:
a) a pilot drill **(1)** for the creation of a pilot bore **(61)** in the form of a blind hole, as preparation for a step bore **(62,63,64)** in the form of a blind hole, to be introduced into a jawbone **(5),** for receiving a dental implant **(4);**
b) a first step drill **(2)** for the enlargement of the existing pilot bore **(61)** into a step bore **(62);**
c) a second step drill **(2)** for the second enlargement of an existing step bore **(62)** into a further enlarged step bore **(63);** and
d) a third step drill **(2)** for the third enlargement of the already twice-enlarged step bore **(63)** into a step bore **(64)** enlarged a final time;
e) the pilot drill **(1)** having:
ea) a pilot tip **(10),** which is arranged at the apical end of the pilot drill **(1)** and has tip cutting edges **(101);**
eb) a pilot guide **(11),** which extends from the pilot tip **(10)** in the direction of the coronal end of the pilot drill **(1);**
ec) a drill neck **(12),** which lies above the pilot guide **(11)** and has a larger drill diameter **(b2)** than the drill diameter **(b1)** of the pilot guide **(11);**
ed) a drill stem **(13),** which lies above the drill neck **(12)** and may be adjoined by a coupling **(14)** as the coronal end;
ee) at least one guide cutting edge **(112),** lying to the side of the pilot guide **(11);**
ef) a step **(124),** as a transition from the pilot guide **(11)** to the drill neck **(12);**
eg) at least one step cutting edge **(125)** at the step **(124);** and
eh) at least one spiral groove **(122)** and an adjacent bevel **(123);** and
f) the step drill **(2)** has:
fa) a step tip **(20),** which is arranged at the apical end of the step drill **(2)** and has tip cutting edges **(201);**
fb) a step guide **(21),** which extends from the step tip **(20)** in the direction of the coronal end of the step drill **(2);**
fc) a drill neck **(22),** which lies above the step guide **(21)** and has a larger drill diameter **(b3,b4,b5)** than the drill diameter **(b2',b3',b4')** of the step guide (**21**);
fd) a drill stem **(23),** which lies above the drill neck **(22)** and may be adjoined by a coupling **(24)** as the coronal end;
fe) at least one guide cutting edge **(212),** lying to the side of the step guide (**21**),
ff) a step **(224),** as a transition from the step guide **(21)** to the drill neck **(22);**
fg) at least one step cutting edge **(225)** at the step **(224);** and
fh) at least one spiral groove **(222)** and an adjacent bevel **(223), characterized in that**
g) on the pilot drill **(1):**
ga) the tip cutting edges **(101)** at the pilot tip **(10)** are sharply formed and center-cutting;
gb) chamfers **(111)** extend from the tip cutting edges **(101)** upward of the pilot guide **(11);**
gc) the step cutting edges **(125)** at the step **(124)** are formed in a cutting manner; and
gd) the guide cutting edges **(112)** are formed in a blunt, non-cutting manner;
h) on the step drill **(2):**
ha) the tip cutting edges **(201)** at the step tip **(20)** are sharply formed;
hb) chamfers **(211)** extend from the tip cutting edges **(201)** upward of the step guide **(21);**
hc) the step cutting edges **(225)** at the step **(224)** are formed in a cutting manner; and
hd) the guide cutting edges **(212)** are formed in a blunt, non-cutting manner;
i) the diameter **(b2')** of the first step drill **(2)** at the step guide **(21)** corresponds to the diameter **(b2)** at the drill neck **(12)** of the pilot drill **(1);** and
j) the diameter (**b3',b4'**) of the second and third step drills **(2)** at the step guide **(21)** corresponds to the diameter **(b3,b4)** at the drill neck **(22)** of the previous first or second step drill **(2),** respectively.

10. The drill set as claimed in claim 9, **characterized in that**, of the pilot drill **(1),** the drill neck **(12)** with the bevel **(123)** is formed in a weakly cutting manner and its pilot guide **(11)** has a length **(11)** in the range from 1.0 mm to 4.0 mm.

11. The drill set as claimed in claim 9 or 10, **characterized in that**
a) the pilot drill **(1)** is formed with two cutting edges and consequently has two tip cutting edges **(101),** two chamfers **(111),** two guide cutting edges **(112),** two spiral grooves **(122),** two bevels **(123)** and two step cutting edges **(125);**
b) the drill neck **(12)** has a length at least equal to the depth of insertion of the implant **(4)** to be applied;
c) the pilot guide **(11)** has a length **(11)** of 3.0 mm;
d) the pilot guide **(11)** has a diameter **(b1)** in the region of 1.5 mm and the drill neck **(12)** has a diameter **(b2)** in the region of 2.0 mm;
e) the tip angle (α) lying between the tip cutting edges **(101)** is less than 90°, preferably lies in the region of 80°;
f) the spiral grooves **(122)** extent continuously from the coronal end of the drill neck **(12)** into the pilot tip **(10),** the spiral grooves **(122)** having at the pilot guide **(11)** only a fraction of their full cross section, as present at the drill neck **(12),** as a result of the smaller diameter **(b1);**
g) a number of visible depth markings **(121)** are provided at equal or unequal intervals on the drill neck **(12);** and
h) the coupling **(14)** adjoining the drill stem **(13)** is a standardized dental coupling.

12. The drill set as claimed in one of claims 9 to 11, **characterized in that**
a) the pilot guide **(11)** with the pilot tip **(10)** are intended to fix the position of the step bore **(62,63,64)** that is to be produced, by introducing a start of a pilot bore **(61)** through the cortical bone **(51)** of the jawbone **(5),** the start comprising the pilot bore guide **(611)** and a pilot bore tip **(610);**
b) the step **(124)** is intended for the purpose of generating a noticeably increased drilling resistance once the cortical bone **(51)** is penetrated, with completion of the pilot bore guide **(611)** and tip **(610),** and use this indication to check the drilling direction **(R)** that has been set up;
c) the blunt guide cutting edges **(112)** make it possible to correct the drilling direction (**R)** within a conical range of correction **(K)** without widening the pilot bore guide **(611);** and
d) the drill neck **(12)** with its dimensioning is intended to create the pilot bore **(61)** with the final depth.

13. The drill set as claimed in claim 9, **characterized in that**, of the step drill **(2),** the drill neck **(22)** with the bevel **(223)** is formed in a weakly cutting manner and its step guide **(21)** has a length (**l2,l3,l4**) in the region of 2.0 mm.

14. The drill set as claimed in claim 9 or 13, **characterized in that**
a) the step drill **(2)** is formed with three cutting edges and consequently has three tip cutting edges **(201),** three chamfers **(211),** three guide cutting edges **(212),** three spiral grooves **(222),** three bevels **(223)** and three step cutting edges **(225);**
b) the drill neck **(22)** has a length at least equal to the depth of insertion of the implant **(4)** to be applied;
c) the step guide **(21)** has a diameter **(b2',b3',b4')** in the region of 2.0 mm, 2.8 mm and 3.5 mm, respectively, and the drill neck **(22)** has a diameter **(b3,b4,b5)** in the region of 2.8 mm, 3.5 mm and 4.3 mm, respectively;
d) the tip angle (β) lying between the tip cutting edges **(201)** is greater than 90°, preferably lies in the region of 120°;
e) the spiral grooves **(222)** extend continuously from the coronal end of the drill neck **(22)** into the step tip **(20),** the spiral grooves **(222)** having at the step guide **(21)** only a fraction of their full cross section, as present at the drill neck **(22),** as a result of the smaller diameter (**b2',b3',b4'**);
f) a number of visible depth markings **(221)** being provided at equal or unequal intervals on the drill neck **(22);** and
g) the coupling **(24)** adjoining the drill stem **(23)** is a standardized dental coupling.

15. The drill set as claimed in one of claims 9, 13 or 14, **characterized in that**
a) the step guide **(21)** with the step tip **(10)** and the blunt guide cutting edges **(212)** is intended for centering the step drill **(2)** when setting it up in the pilot bore **(61)** or step bore **(62,63)** and guiding it in a centered manner when advancing along the pilot bore **(61)** or the step bore **(62,63);** and
b) the step **(224)** with the step cutting edges **(225)** is intended for widening the pilot bore **(61)** with the diameters (**d1/d2**) to the diameters **(d2/d3)** or for widening the step bore **(62,63)** with the diameters **(d2/d3,d3/d4)** to the diameters **(d3/d4,d4/d5)** of the step bore **(63,64).**

## Revendications

1. Foret pilote **(1)** pour la préparation d'un forage étagé **(62,63,64)** en forme de trou borgne à pratiquer dans un os maxillaire **(5)** en vue de la pose d'un implant dentaire **(4),** avec:
a) une pointe pilote **(10),** qui est disposée à l'extrémité apicale du foret pilote **(1)** et qui comporte des tranchants de pointe **(101);**
b) un guide pilote **(11),** qui s'étend à partir de la pointe pilote **(10)** en direction de l'extrémité coronale du foret pilote **(1);**
c) un col de foret **(12),** qui est situé au-dessus du guide pilote **(11)** et qui présente un plus grand diamètre de foret **(b2)** que le diamètre de foret **(b1)** du guide pilote **(11);**
d) un corps de foret, **(13)** situé au-dessus du col de foret **(12),** auquel un couplage **(14)** peut se raccorder en guise d'extrémité coronale;
e) au moins un tranchant de guidage **(112)** situé latéralement au guide pilote (**11**);
f) un étage **(124),** formant la transition du guide pilote **(11)** au col de foret **(12);**
g) au moins un tranchant étagé **(125)** sur l'étage **(124);** et
h) au moins une rainure spirale **(122)** et un chanfrein **(123)** adjacent à celle-ci, **caractérisé en ce que**
i) les tranchants de pointe **(101)** sur la pointe pilote **(10)** sont affûtés et sont coupants au centre;
j) des entailles polies **(111)** s'étendent à partir des tranchants de pointe **(101)** vers le haut du guide pilote **(11);**
k) les tranchants étagés **(125)** sur l'étage **(124)** sont coupants; et
l) les tranchants de guidage **(112)** sont émoussés et non coupants.

2. Foret pilote **(1)** selon la revendication 1, **caractérisé en ce que**
a) le col de foret **(12)** avec le chanfrein **(123)** est faiblement coupant; et
b) le guide pilote **(11)** présente une longueur **(l1)** comprise dans la plage de 1,0 mm à 4,0 mm.

3. Foret pilote **(1)** selon la revendication 1 ou 2, **caractérisé en ce que**
a) le foret pilote **(1)** est réalisé à deux tranchants et présente de ce fait respectivement deux tranchants de pointe **(101),** deux entailles polies **(111),** deux tranchants de guidage **(112),** deux rainures spirales **(122),** deux chanfreins **(123)** et deux tranchants étagés **(125);**
b) le col de foret **(12)** présente au moins la longueur de la profondeur d'insertion de l'implant à appliquer **(4);**
c) le guide pilote **(11)** a la longueur **(11)** de 3,0 mm;
d) le guide pilote **(11)** présente le diamètre **(b1)** de l'ordre de 1,5 mm et le col de foret **(12)** présente le diamètre **(b2)** de l'ordre de 2,0 mm;
e) l'angle de pointe (α) situé entre les tranchants de pointe **(101)** est inférieur à 90°, et est de préférence de l'ordre de 80°;
f) les rainures spirales **(122)** s'étendent de façon continue depuis l'extrémité coronale du col de foret **(12)** jusque dans la pointe pilote **(10),** les rainures spirales **(122)** ne présentant plus, au guide pilote **(11),** en raison du diamètre plus petit **(b1),** qu'une partie de leur pleine section transversale, telle qu'elle est présente au col de foret **(12);**
g) plusieurs marquages de profondeur visibles **(121)** sont appliqués sur le col de foret **(12),** à des distances égales ou inégales; et
h) le couplage **(14)** se raccordant au corps de foret **(13)** est un couplage dentaire normalisé.

4. Foret pilote **(1)** selon l'une quelconque des revendications 1 à 3, **caractérisé en ce que**
a) le guide pilote **(11)** avec la pointe pilote **(10)** est destiné à fixer la position du forage étagé à produire **(62,63,64)** avec la réalisation d'une amorce d'un forage pilote **(61)** à travers la corticale **(51)** de l'os maxillaire **(5),** l'amorce se composant d'un guide de forage pilote **(611)** et d'une pointe de forage pilote **(610);**
b) l'étage **(124)** est destiné, une fois la corticale **(51)** traversée avec finition du guide de forage pilote **(611)** et de la pointe de forage pilote **(610),** à produire une résistance au forage nettement accrue et à vérifier avec cet indice la direction de forage amorcée **(R);**
c) les tranchants de guidage émoussés **(112)** permettent, sans élargissement du guide de forage pilote **(611),** de corriger la direction de forage **(R)** à l'intérieur d'une plage de correction de forme conique **(K);** et
d) le col de foret **(12)** est destiné, avec son dimensionnement, à fixer le forage pilote **(61)** à sa profondeur définitive.

5. Foret étagé **(2)** pour l'agrandissement d'un forage pilote en forme de trou borgne **(61)** existant dans un os maxillaire **(5)** en un forage étagé **(62)** ou pour l'agrandissement d'un forage étagé existant **(62,63)** en un forage étagé encore agrandi **(63,64)** en vue de la pose d'un implant dentaire **(4),** avec:
a) une pointe étagée **(20),** qui est disposée à l'extrémité apicale du foret étagé **(2)** et qui comporte des tranchants de pointe **(201);**
b) un guide étagé **(21),** qui s'étend à partir de la pointe étagée **(20)** en direction de l'extrémité coronale du foret étagé **(2);**
c) un col de foret **(22),** qui est situé au-dessus du guide étagé **(21)** et qui présente un plus grand diamètre de foret **(b3,b4,b5)** que le diamètre de foret **(b2',b3',b4')** du guide étagé **(21);**
d) un corps de foret **(23)** situé au-dessus du col de foret **(22),** auquel un couplage **(24)** peut se raccorder en guise d'extrémité coronale;
e) au moins un tranchant de guidage **(212)** situé latéralement au guide étagé (**21**);
f) un étage **(224),** formant la transition du guide étagé **(21)** au col de foret **(22);**
g) au moins un tranchant étagé **(225)** sur l'étage **(224);** et
h) au moins une rainure spirale **(222)** et un chanfrein **(223)** adjacent à celle-ci, **caractérisé en ce que**
i) les tranchants de pointe **(201)** sur la pointe étagée **(20)** sont affûtés;
j) des entailles polies **(211)** s'étendent à partir des tranchants de pointe **(201)** vers le haut du guide étagé **(21);**
k) les tranchants étagés **(225)** sur l'étage **(224)** sont coupants; et
l) les tranchants de guidage **(212)** sont émoussés et non coupants.

6. Foret étagé **(2)** selon la revendication 5, **caractérisé en ce que**
a) le col de foret **(22)** avec le chanfrein **(223)** est faiblement coupant; et
b) le guide étagé **(21)** présente une longueur (**l2,l3,l4**) de l'ordre de 2,0 mm.

7. Foret étagé **(2)** selon la revendication 5 ou 6, **caractérisé en ce que**
a) le foret étagé **(2)** est réalisé à trois tranchants et présente de ce fait respectivement trois tranchants de pointe **(201),** trois entailles polies **(211),** trois tranchants de guidage **(212),** trois rainures spirales **(222),** trois chanfreins **(223)** et trois tranchants étagés **(225);**
b) le col de foret **(22)** présente au moins la longueur de la profondeur d'insertion de l'implant à appliquer **(4);**
c) le guide étagé **(21)** présente le diamètre **(b2',b3',b4')** de l'ordre de 2,0 mm, 2,8 mm, 3,5 mm et le col de foret **(22)** présente le diamètre **(b3,b4,b5)** de l'ordre de 2,8 mm, 3,5 mm, 4,3 mm;
d) l'angle de pointe (β) situé entre les tranchants de pointe **(201)** est supérieur à 90°, et est de préférence de l'ordre de 120°;
e) les rainures spirales **(222)** s'étendent de façon continue depuis l'extrémité coronale du col de foret **(22)** jusque dans la pointe étagée **(20),** les rainures spirales **(222)** ne présentant plus, au guide étagé **(21),** en raison du diamètre plus petit (**b2',b3',b4'**), qu'une partie de leur pleine section transversale, telle qu'elle est présente au col de foret **(22);**
f) plusieurs marquages de profondeur visibles **(221)** sont appliqués sur le col de foret **(22),** à des distances égales ou inégales; et
g) le couplage **(24)** se raccordant au corps de foret **(23)** est un couplage dentaire normalisé.

8. Foret étagé **(2)** selon l'une quelconque des revendications 5 à 7, **caractérisé en ce que**
a) le guide étagé **(21)** avec la pointe étagée **(10)** et les tranchants de guidage émoussés **(212)** est destiné à centrer le foret étagé **(2)** lors du placement dans le forage pilote **(61)** ou le forage étagé **(62,63)** et le guider de façon centrée lors de l'avancement le long du forage pilote **(61)** ou du forage étagé **(62,63);** et
b) l'étage **(224)** avec les tranchants étagés **(225)** est destiné à élargir le forage pilote **(61)** avec les diamètres (**d1/d2**) aux diamètres **(d2/d3)** ou d'élargir le forage étagé **(62,63)** avec les diamètres **(d2/d3, d3/d4)** aux diamètres **(d3/d4, d4/d5**) du forage étagé (**63,64**)

9. Ensemble de forets se composant de:
a) un foret pilote **(1)** pour la réalisation d'un forage pilote en forme de trou borgne **(61)** comme préparation pour un forage étagé en forme de trou borgne **(62,63,64)** à pratiquer dans un os maxillaire **(5)** en vue de la pose d'un implant dentaire **(4);**
b) un premier foret étagé **(2)** pour l'agrandissement du forage pilote existant **(61)** en un forage étagé **(62);**
c) un deuxième foret étagé **(2)** pour le deuxième agrandissement d'un forage étagé existant **(62)** en un forage étagé encore agrandi **(63);** et
d) un troisième foret étagé **(2)** pour le troisième agrandissement du forage étagé déjà agrandi deux fois **(63)** en un forage étagé agrandi une dernière fois **(64);** dans lequel
e) le foret pilote **(1)** présente:
ea) une pointe pilote **(10),** qui est disposée à l'extrémité apicale du foret pilote **(1**) et qui comporte des tranchants de pointe **(101);**
eb) un guide pilote **(11),** qui s'étend à partir de la pointe pilote **(10)** en direction de l'extrémité coronale du foret pilote **(1);**
ec) un col de foret **(12),** qui est situé au-dessus du guide pilote **(11)** et qui présente un plus grand diamètre de foret **(b2)** que le diamètre de foret **(b1)** du guide pilote **(11);**
ed) un corps de foret **(13)** situé au-dessus du col de foret **(12),** auquel un couplage **(14)** peut se raccorder en guise d'extrémité coronale;
ee) au moins un tranchant de guidage **(112)** situé latéralement au guide pilote (**11**);
ef) un étage **(124),** formant la transition du guide pilote **(11)** au col de foret **(12);**
eg) au moins un tranchant étagé **(125)** sur l'étage **(124);** et
eh) au moins une rainure spirale (**122**) et un chanfrein (**123**) adjacent à celle-ci; et
f) le foret étagé (**2**) présente:
fa) une pointe étagée (**20**), qui est disposée à l'extrémité apicale du foret étagé (**2**) et qui comporte des tranchants de pointe (**201**);
fb) un guide étagé (**21**), qui s'étend à partir de la pointe étagée (**20**) en direction de l'extrémité coronale du foret étagé (**2**);
fc) un col de foret (**22**), qui est situé au-dessus du guide étagé (**21**) et qui présente un plus grand diamètre de foret (**b3**,**b4**,**b5**) que le diamètre de foret (**b2'**,**b3'**,**b4'**) du guide étagé (**21**);
fd) un corps de foret (**23**) situé au-dessus du col de foret (**22**), auquel un couplage (**24**) peut se raccorder en guise d'extrémité coronale;
fe) au moins un tranchant de guidage (**212**) situé latéralement au guide étagé (**21**);
ff) un étage (**224**), formant la transition du guide étagé (**21**) au col de foret (**22**);
fg) au moins un tranchant étagé (**225**) sur l'étage (**224**); et
fh) au moins une rainure spirale (**222**) et un chanfrein (**223**) adjacent à celle-ci,
**caractérisé en ce que**
g) sur le foret pilote (**1**):
ga) les tranchants de pointe (**101**) sur la pointe pilote (**10**) sont affûtés et sont coupants au centre;
gb) des entailles polies (**111**) s'étendent à partir des tranchants de pointe (**101**) vers le haut du guide pilote (**11**);
gc) les tranchants étagés (**125**) sur l'étage (**124**) sont coupants; et
gd) les tranchants de guidage (**112**) sont émoussés et non coupants;
h) sur le foret étagé (**2**):
ha) les tranchants de pointe (**201**) sur la pointe étagée (**20**) sont affûtés;
hb) des entailles polies (**211**) s'étendent à partir des tranchants de pointe (**201**) vers le haut du guide étagé (**21**);
hc) les tranchants étagés (**225**) sur l'étage (**224**) sont coupants; et
hd) les tranchants de guidage (**212**) sont émoussés et non coupants;
i) le diamètre (**b2**') du premier foret étagé (**2**) au guide étagé (**21**) correspond au diamètre (**b2**) au col de foret (**12**) du foret pilote (**1**); et
j) le diamètre (**b3**',**b4**') du deuxième ou du troisième foret étagé (**2**) au guide étagé (**21**) correspond au diamètre (**b3**,**b4**) au col de foret (**22**) du premier ou du deuxième foret étagé antérieur (**2**).

10. Ensemble de forets selon la revendication 9, **caractérisé en ce que** dans le foret pilote (**1**) le col de foret (**12**) avec le chanfrein (**123**) est faiblement coupant et son guide pilote (**11**) présente une longueur (**I1**) comprise dans la plage de 1,0 mm à 4,0 mm.

11. Ensemble de forets selon la revendication 9 ou 10, **caractérisé en ce que**
a) le foret pilote (**1**) est réalisé à deux tranchants et présente de ce fait respectivement deux tranchants de pointe (**101**), deux entailles polies (**111**), deux tranchants de guidage (**112**), deux rainures spirales (**122**), deux chanfreins (**123**) et deux tranchants étagés (**125**);
b) le col de foret (**12**) présente au moins la longueur de la profondeur d'insertion de l'implant à appliquer (**4**);
c) le guide pilote (**11**) a la longueur (**I1**) de 3,0 mm;
d) le guide pilote (**11**) présente le diamètre (**b1**) de l'ordre de 1,5 mm et le col de foret (**12**) présente le diamètre (**b2**) de l'ordre de 2,0 mm;
e) l'angle de pointe (α) situé entre les tranchants de pointe (**101**) est inférieur à 90°, et est de préférence de l'ordre de 80°;
f) les rainures spirales (**122**) s'étendent de façon continue depuis l'extrémité coronale du col de foret (**12**) jusque dans la pointe pilote (**10**), les rainures spirales (**122**) ne présentant plus, au guide pilote (**11**), en raison du diamètre plus petit (**b1**), qu'une partie de leur pleine section transversale, telle qu'elle est présente au col de foret (**12**);
g) plusieurs marquages de profondeur visibles (**121**) sont appliqués sur le col de foret (**12**), à des distances égales ou inégales; et
h) le couplage (**14**) se raccordant au corps de foret (**13**) est un couplage dentaire normalisé.

12. Ensemble de forets selon l'une quelconque des revendications 9 à 11, **caractérisé en ce que**
a) le guide pilote (**11**) avec la pointe pilote (**10**) est destiné à fixer la position du forage étagé à produire (**62**,**63**,**64**) avec la réalisation d'une amorce d'un forage pilote (**61**) à travers la corticale (**51**) de l'os maxillaire (**5**), l'amorce se composant d'un guide de forage pilote (**611**) et d'une pointe de forage pilote (**610**);
b) l'étage (**124**) est destiné, une fois la corticale (**51**) traversée avec finition du guide de forage pilote (**611**) et de la pointe de forage pilote (**610**), à produire une résistance au forage nettement accrue et à vérifier avec cet indice la direction de forage amorcée (**R**);
c) les tranchants de guidage émoussés (**112**) permettent, sans élargissement du guide de forage pilote (**611**), de corriger la direction de forage (**R**) à l'intérieur d'une plage de correction de forme conique (**K**); et
d) le col de foret (**12**) est destiné, avec son dimensionnement, à fixer le forage pilote (**61**) à sa profondeur définitive.

13. Ensemble de forets selon la revendication 9, caractérisé en ce' que dans le foret étagé (**2**) le col de foret (**22**) avec le chanfrein (**223**) est faiblement coupant et son guide étagé (**21**) présente une longueur (**I2**,**I3**,**I4**) de l'ordre de 2,0 mm.

14. Ensemble de forets selon la revendication 9 ou 13, **caractérisé en ce que**
a) le foret étagé (**2**) est réalisé à trois tranchants et présente de ce fait respectivement trois tranchants de pointe (**201**), trois entailles polies (**211**), trois tranchants de guidage (**212**), trois rainures spirales (**222**), trois chanfreins (**223**) et trois tranchants étagés (**225**);
b) le col de foret (**22**) présente au moins la longueur de la profondeur d'insertion de l'implant à appliquer (**4**);
c) le guide étagé (**21**) présente le diamètre (**b2'**,**b3'**,**b4'**) de l'ordre de 2,0 mm, 2,8 mm, 3,5 mm et le col de foret (**22**) présente le diamètre (**b3**,**b4**,**b5**) de l'ordre de 2,8 mm, 3,5 mm, 4,3 mm;
d) l'angle de pointe (β) situé entre les tranchants de pointe (**201**) est supérieur à 90°, et est de préférence de l'ordre de 120°;
e) les rainures spirales (**222**) s'étendent de façon continue depuis l'extrémité coronale du col de foret (**22**) jusque dans la pointe étagée (**20**), les rainures spirales (**222**) ne présentant plus, au guide étagé (**21**), en raison du diamètre plus petit (**b2'**,**b3'**,**b4'**), qu'une partie de leur pleine section transversale, telle qu'elle est présente au col de foret (**22**);
f) plusieurs marquages de profondeur visibles (**221**) sont appliqués sur le col de foret (**22**), à des distances égales ou inégales; et
g) le couplage (**24**) se raccordant au corps de foret (**23**) est un couplage dentaire normalisé.

15. Ensemble de forets selon l'une quelconque des revendications 9, 13 ou 14, **caractérisé en ce que**
a) le guide étagé (**21**) avec la pointe étagée (**10**) et les tranchants de guidage émoussés (**212**) est destiné à centrer le foret étagé (**2**) lors du placement dans le forage pilote (**61**) ou le forage étagé (**62**,**63**) et le guider de façon centrée lors de l'avancement le long du forage pilote (**61**) ou du forage étagé (**62**,**63**); et
b) l'étage (**224**) avec les tranchants étagés (**225**) est destiné à élargir le forage pilote (**61**) avec les diamètres (**d1**/**d2**) aux diamètres (**d2**/**d3**) ou d'élargir le forage étagé (**62**,**63**) avec les diamètres (**d2**/**d3**, **d3**/**d4**) aux diamètres (**d3**/**d4**, **d4**/**d5**) du forage étagé (**63**,**64**).
